# EUROPEAN PATENT SPECIFICATION

(11) **EP 4 128 271 B1**
(45) Date of publication and mention of the grant of the patent: **25.02.2026**
(21) Application number: 21776494.3
(22) Date of filing: 23.03.2021
(51) Int. Cl.: G16H 50/20, G06N 20/00, G16H 10/60, G16H 15/00, G16H 50/50, G16H 50/70, G16H 80/00

(54) **FULLY AUTONOMOUS MEDICAL SOLUTION (MYDOCTOR)**
VOLLAUTONOME MEDIZINISCHE LÖSUNG (MYDOCTOR)
SOLUTION MÉDICALE COMPLÈTEMENT AUTONOME (MYDOCTOR)

(30) Priority: 23.03.2020 US 202062993437 P; 17.06.2020 US 202016904219
(43) Date of publication of application: 08.02.2023
(73) Proprietor: Al-Sinan, Mazen, A., Dhahran, 31311 (SA); Kuo, Ho-Hsun, David, Flagstaff, AZ 86001 (US)
(72) Inventor: Al-Sinan, Mazen, A., Dhahran, 31311 (SA); Kuo, Ho-Hsun, David, Flagstaff, AZ 86001 (US)
(74) Representative: Puchberger & Partner Patentanwälte
(86) International application number: PCT/US2021/023661
(87) International publication number: WO 2021/195072

(56) References cited:
- US-A1- 2017 098 051
- US-A1- 2017 132 367
- US-A1- 2018 068 082
- US-A1- 2018 101 652
- US-A1- 2019 139 648
- US-A1- 2019 244 683
- US-A1- 2020 029 837
- RAFAILIDIS DIMITRIOS, MANOLOPOULOS YANNIS: "Can Virtual Assistants Produce Recommendations?", PROCEEDINGS OF THE 9TH INTERNATIONAL CONFERENCE ON WEB INTELLIGENCE, MINING AND SEMANTICS , WIMS2019, 26 June 2019 (2019-06-26), pages 1 - 6, XP055864019, Retrieved from the Internet <URL:https:/Idl.acm.org/doi/pdf/10.1145/3326467.3326468> [retrieved on 20210406]

## Description

### CROSS-REFERENCES TO RELATED APPLICATIONS

This application claims benefit of U.S. Provisional Patent Application No. 62/993,437, filed March 23, 2020 and U.S. Patent Application No. 16/904,219, filed June 17, 2020.

### FIELD OF THE INVENTION

The present invention is directed to the integration of various services under one platform and mimicking a role of a physician within a personal computing device.

### BACKGROUND OF THE INVENTION

The present invention is in the technical field of intelligent medical automation. More particularly, the present invention is in the technical field of autonomous medical solutions to diagnosis and provide treatment to an individual. There are many challenges with the current practices for seeking medical care. When an individual experiences an abnormality in their health condition, one of the following scenarios could take place:

1. Seeing a physician while the condition might be minor and temporary and doesn't require seeing a physician or the problem could be cured by taking over-the-counter drugs or by non-drug treatment. However, identifying the suitable over-the-counter drug is challenging.

2. Ignoring the symptoms or taking over-the-counter drugs while there is a genuine need to see a physician, considering the fact that many people avoid seeing physicians for various reasons; such as financial expenses, time constraint, or fear from seeing a physician.

As a result, seeing a physician while there is no need is a waste of resources, while avoiding seeing one when it is necessary could aggravate the condition and endanger the life of the patient.

Furthermore, many individuals live alone and in case of a need for medical care, especially when they are sick, might find ordering and collecting an over-the-counter drug, identifying appropriate medical centers, setting up an appointment with a physician, or, in severe cases, calling for an ambulance could be challenging.

Another issue is that medical illness could occur at any time while physicians are only available during working hours unless the patient decides to go to the emergency room. Going to the emergency in many cases could be avoided; the patient may simply need temporary relief or someone to advise him/her whether there is an urgent need to go to the emergency or not.

For example, elderly people and those who might be susceptible to heart attacks or a crucial need for emergency medical intervention, should be monitored around the clock by someone such as a home nurse or an accompanying party who should call for an ambulance in case of any abnormalities in vital signs that necessitate urgent medical intervention.

Furthermore, physicians usually encounter challenges in diagnosing diseases since many of them have similar or nonspecific signs and symptoms. For instance, red spots on the skin could be a sign of a kidney problem. A physician could diagnose a condition based on experience or results of many tests and examinations which might be unnecessary. Physicians depend on their experience and the results of tests and examinations to diagnose any medical condition. However, Artificial Intelligence (A.I.) and Machine Learning (M.L.) could outperform physicians in diagnosing a patient's condition provided the results of tests and examinations are fed.

On the other hand, a patient's medical historical record is important for the physician to diagnose and to prescribe the appropriate treatment. Reviewing the medical history is time consuming to the physician and much important information relevant to the patient's condition could be overlooked. In case of emergency, the physician would take actions without reviewing the medical records of the patient. Similarly, when the medical records are unavailable, they might request unnecessary tests or proceed with prescribing a treatment regardless of the patient's medical historical record. A physician could also prescribe a medicine hoping that it will resolve the problem and in the follow up appointment which is usually after one week the physician could discover that the medicine is not working and has to find an alternative medication or treatment.

In some cases, a patient might need temporary home nursing care such as in case of severe flu where the patient's condition does not require admission to the hospital but still requires someone to help them, such as a certified nurse if they live alone. Finding a freelance nurse within a short duration in these cases could be difficult.

All the above challenges have substantial impacts from wasting resources by taking unnecessary tests to seeing a physician while the problem could be resolved with an over-the-counter drug to exposing the patient to a fatal risk or aggravation of the patient health condition because of the delay in seeking the medical care while there is an imminent need for a medical intervention.

In a situation similar to Covid-19 pandemic, the proposed invention could sustain medical services for most routine medical care services, especially in the case of conditions that do not require performing medical procedures. The stretched medical resources could focus on the pandemic while the patient could still receive medical services while avoiding going to the medical centers that could be risky because of the pandemic. It could also provide thousands of self-isolated patients with necessary medical consultations on the spot.

A.I. has been previously utilized in diagnosis and health services, but the fundamental challenges in medical services have not been surmounted proportionally to the advancement in A.I. applications in general and in health services in particular. There is a potential to enhance quality and optimization of resources in medical services throughout by applying A.I. technology in an integrated manner. Technologies such as Blockchain, Machine Learning (M.L.), Internet of Things (loT), Natural Language Processing (NLP) and Data Science could additionally be integrated and employed to have one unified synchronized MyDoctor platform that provides a full medical solution to individuals.

The present invention utilizes artificial intelligence, machine learning, medical history and natural language for interaction with the system to mimic the role of the physician to provide medical treatment without the presence of the physician

Prior art document US2019244683 discloses a computer program product for assembling a database comprising electronic medical records (EMRs). An EMR comprises at least one active diagnosis module (ADM). The database is searchable based on at least some ADM content. Diagnostic tests and treatments may be suggested.

### BRIEF SUMMARY OF THE INVENTION

The invention is set out in the appended claims.

### SUMMARY OF RELATED DISCLOSURE

It is an objective of the present invention to provide systems that allow for the integration of various services under one platform and mimicking a role of a physician within a personal computing device, as specified in the independent claims. Embodiments of the invention are given in the dependent claims. Embodiments of the present invention can be freely combined with each other if they are not mutually exclusive.

The present invention features a MyDoctor platform. The platform may comprise an external communicator module. The external communicator module may comprise a data encryption module for accepting data from a plurality of sources and encrypting the data into a standard format. The data encryption module may accept information from a user, or from a plurality of external medical sources. The external communicator module may further comprise a data compression module for compressing and transmitting encrypted data. The data compression module may transmit data to the plurality of external medical sources or to an external storage component. The plurality of external medical sources may comprise a hospital, a clinic, a lab, an emergency center, and a pharmacy.

The MyDoctor platform may additionally feature a diagnosis module. The diagnostic module may comprise a database of disease symptoms mapped to possible causes. The diagnostic module may comprise instructions for accessing the database of disease symptoms and accepting a list of user symptoms from the user. The diagnostic module may further comprise generating a table to map each user symptom to all possible causes for each symptom and generating a second table displaying all possible causes that share every user symptom. The diagnostic module may further comprise accepting data from a database of user medical records and generating a probability table ranking all possible causes based on the probability of the presence of each possible cause. Finally, the diagnostic module may determine a treatment for the possible causes based on information from the database of medical records. In some embodiments, the diagnostic module may additionally request tests from the user to better determine the possible cause.

The MyDoctor platform of the present invention may additionally feature a case monitoring module. The case monitoring module may comprise instructions for monitoring the user's health and recovery as the user carries out a treatment determined by the diagnostic module. The case monitoring module may further comprise instructions for reminding the user to carry out a prescription related to the treatment, comparing an expected recovery time to an actual recovery time to determine if a secondary course of action is necessary, adjusting the prescription as the secondary course of action, and adjusting the expected recovery time based on the secondary course of action.

One of the unique and inventive technical features of the present invention is the encryption of data from both the user and a plurality of external medical sources into a standard format that can be used interchangeably by both parties. Without wishing to limit the invention to any theory or mechanism, it is believed that the technical feature of the present invention advantageously provides for a comprehensive source of a user's medical history and conditions to allow for more efficient diagnosis of user issues, and allows the MyDoctor platform to send diagnosis information to the external medical sources for later archiving and use. The autonomous aspect of the feature provides for a time and resource efficient manner of compiling and transmitting the user's medical history. None of the presently known prior references or work has the unique inventive technical feature of the present invention. Furthermore, the feature of the present invention is counterintuitive. The reason that it is counterintuitive is because the feature contributed to a surprising result. One skilled in the art would expect the conversion of data to and from a plurality of different formats between the user and the plurality of external medical sources to require a large amount of computation and time to execute properly, making it more efficient overall to simply view, compile, and transmit the data manually. Surprisingly, the present invention is capable of viewing, compiling, and transmitting data between a plurality of sources and a plurality of formats efficiently by generating a script for each encountered format to quickly execute for future instances of the same formats. Thus, the feature of the present invention contributed to a surprising result and is counterintuitive.

Another unique and inventive technical feature of the present invention is the use of a database of disease symptoms and a user's medical history to automatically diagnose a medical issue and generate treatment options for the diagnosis. Without wishing to limit the invention to any theory or mechanism, it is believed that the technical feature of the present invention advantageously provides for an efficient, comprehensive, and accurate form of automatic diagnosis and treatment that the user can execute from the comfort of home. None of the presently known prior references or work has the unique inventive technical feature of the present invention. Furthermore, the feature of the present invention is counterintuitive. The reason that it is counterintuitive is because it contributed to a surprising result. For example, one skilled in the art would expect that the system would be unable to handle a situation in which a user reports symptom that correspond to more than one disease, even given the information from the disease database and the user's medical records. Surprisingly, this situation is overcome by the MyDoc platform through the use of a probability table that lists the most likely matches for the user's condition through the use of the user's medical history and additional tests requested by the diagnostic module, providing for an accurate diagnosis a mass majority of the time. Thus, the feature of the present invention contributed to a surprising result and is counterintuitive.

Another unique and inventive technical feature of the present invention is the prediction of recovery time for a treatment and the adjustment of the recovery time upon application of a secondary course of action. Without wishing to limit the invention to any theory or mechanism, it is believed that the technical feature of the present invention advantageously provides for an efficient and accurate way for the user to plan out their recovery time and keep track of whether or not their treatment is progressing at the correct pace. None of the presently known prior references or work has the unique inventive technical feature of the present invention. Furthermore, the feature of the present invention is counterintuitive. The reason that it is counterintuitive is because it contributed to a surprising result. For example, one skilled in the art would expect the planned recovery time to be entirely inaccurate in certain cases, such as when a medication that a user is already taking quickens or slows recovery from an average recovery time in a database. Surprisingly, the present invention is able to predict recovery times for a specific user accurately through the use of active monitoring and calculations that personalize recovery times to the user, resulting in a more accurate prediction. Thus, the feature of the present invention contributed to a surprising result and is counterintuitive.

Any feature or combination of features described herein are included within the scope of the present invention provided that the features included in any such combination are not mutually inconsistent as will be apparent from the context, this specification, and the knowledge of one of ordinary skill in the art. Additional advantages and aspects of the present invention are apparent in the following detailed description and claims.

### BRIEF DESCRIPTION OF THE SEVERAL VIEWS OF THE DRAWING(S)

The features and advantages of the present invention will become apparent from a consideration of the following detailed description presented in connection with the accompanying drawings in which:
FIG. 1 shows a schematic of an external communicator module of the present invention.
FIG. 2 shows a schematic of a diagnosis module of the present invention using the medical records and user symptoms to generate a list of possible causes for the user symptoms.
FIG. 3 shows a schematic of the diagnosis module of the present invention using a database of disease information to create a plurality of tables and generate a list of possible causes for the user symptoms.
FIG. 4 shows a schematic of the MyDoctor platform of the present invention.
FIG. 5 shows a block diagram of the subject matter illustrating a general scheme of the Fully Autonomous Medical Solution for Individuals.
FIG. 6 shows a block diagram of the User Account Module and its integration with the whole invention.

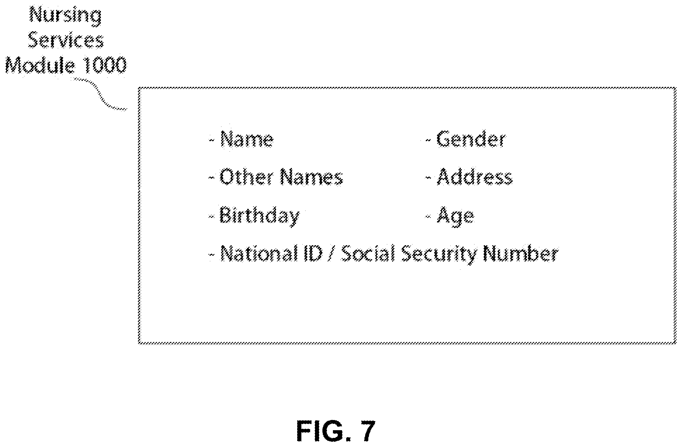

FIG. 7 shows a block diagram of the General Information.

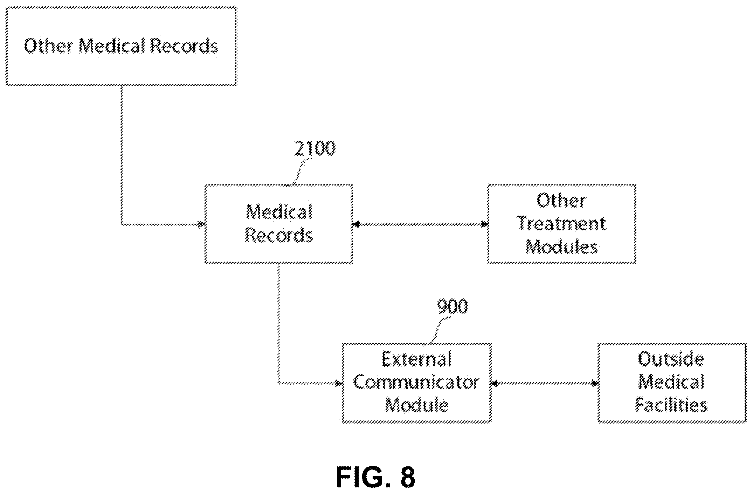

FIG. 8 shows a block diagram of the Medical Records.

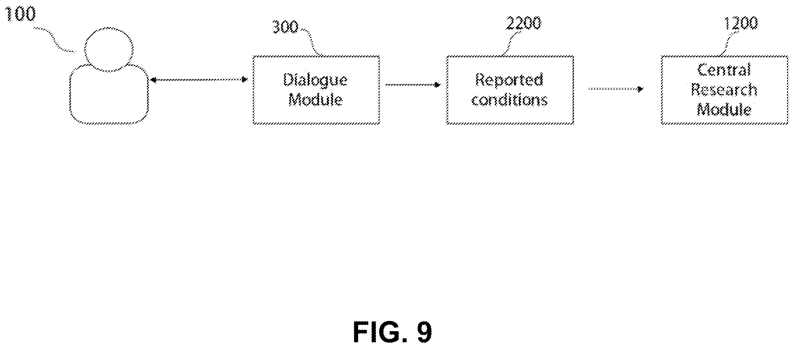

FIG. 9 shows a block diagram of the Reported conditions.

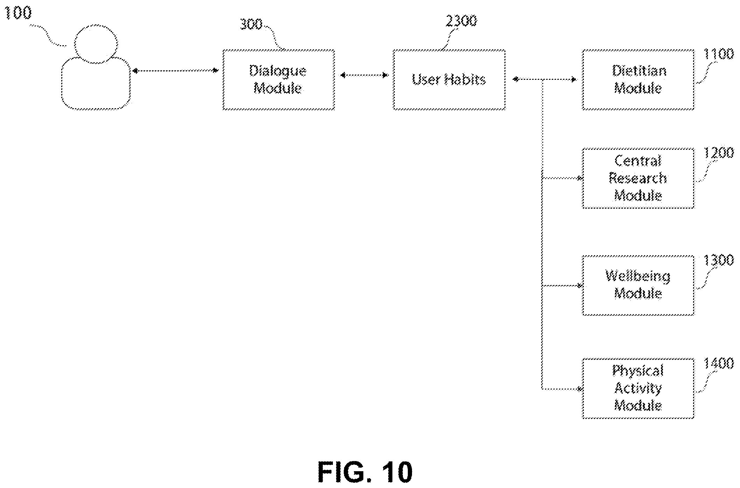

FIG. 10 shows a block diagram of the User Habits.

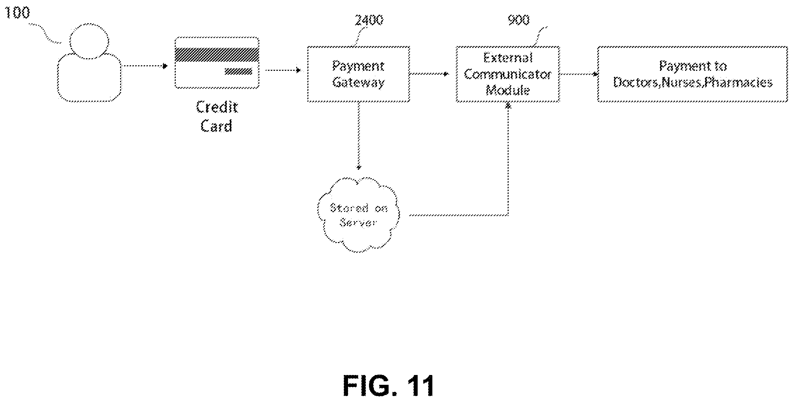

FIG. 11 shows a block diagram of the Payment Gateway.
FIG. 12 shows a block diagram of the Dialogue Module.

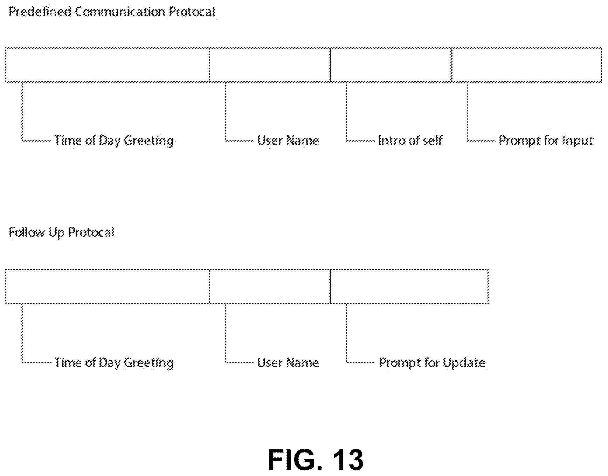

FIG. 13 shows a block diagram of the sample greetings of the Dialogue Module.
FIG. 14 shows a block diagram of the Diagnostic Module.
FIG. 15 shows a block diagram of the property analysis of the Diagnostic Module.
FIG. 16 shows a block diagram of the Test Analyzer Module.
FIG. 17 shows a block diagram of the Treatment Module.

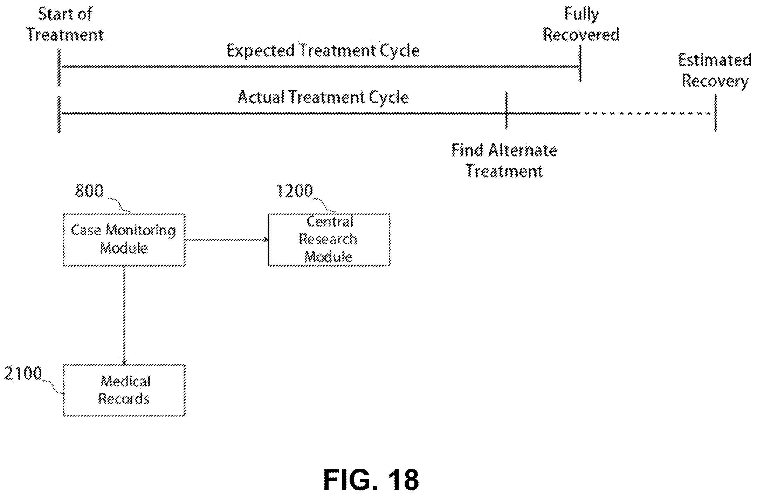

FIG. 18 shows a block diagram of the Case Monitoring Module.
FIG. 19 shows a block diagram of the External Communicator Module.
FIG. 20 shows a block diagram of the Nursing Services Module.
FIG. 21 shows a block diagram of the Dietitian Module.
FIG. 22 shows a block diagram of the Central Research Module.
FIG. 23 shows a block diagram of the Wellbeing Module.
FIG. 24 shows a block diagram of the Physical Activity Module.
FIG. 25 shows a block diagram of the Breathing Apparatus Module.
FIG. 26 shows a block diagram of the Defibrillator Module.
FIG. 27 shows a block diagram of the Psychological Therapy Module.
FIG. 28 shows a block diagram of drone interactions.

### DETAILED DESCRIPTION OF THE INVENTION

Following is a list of elements corresponding to a particular element referred to herein:
100 user
200 user account module
300 dialogue module
400 diagnostic module
410 first table
420 second table
430 probability table
450 list of possible causes
475 treatment options
500 test analyzer module
600 MyDoctor platform
700 treatment module
800 case monitoring module
900 external communicator module
910 data encryption module
920 data compression module
930 plurality of external medical sources
1000 nursing services module
1100 dietician module
1200 central research module
1300 wellbeing module
1400 physical activity module
1500 breathing apparatus module
1600 defibrillator module
1700 psychological therapy module
2000 general information
2100 medical records
2200 reported condition
2300 user habits
2400 payment gateway
9000 external storage component
9100 drone

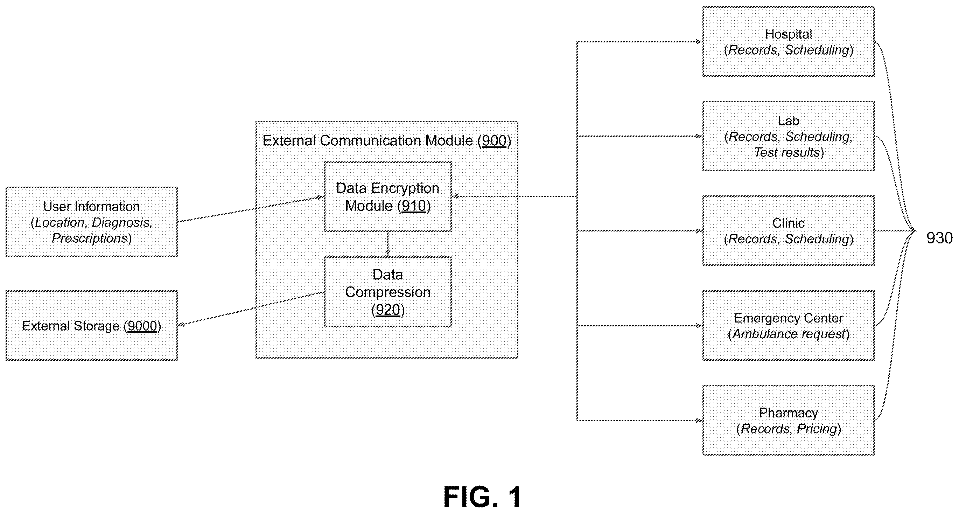

Referring now to FIG. 1, the present invention features a virtual physician (MyDoctor) platform (600) for compiling a user's medical history from a plurality of sources. In some embodiments, the platform may comprise an external communicator module (930) for gathering information pertaining to the user's medical history from a plurality of sources. The external communicator module (900) may comprise a data encryption module (910) with instructions for accepting data from a source, and encrypting the data into a standard format. The data encryption module (910) is capable of accepting data directly from the user, or from a plurality of external medical sources (930). In some embodiments, the process is for a person to process the data for each hospital/format/medical group, and the platform may learn from each format and process the conversion procedure as a script and repeat the process for all future information of the same format. For example, if data format A needs to be converted to data format Z, a person or the system utilizing machine learning may process the data to pull out the personal information, medical records etc. The location of the information and how it is stored as data format Å may be stored and for all future data format A can be converted the same way. This process may be repeated for all other formats. For image data, the images may be exported from the source and processed by the platform by using machine learning to read and analyze the images. Images may be formatted into Nifti or Diacom.

The external communicator module (900) may further comprise a data compression module (920) having instructions for accepting encrypted data from the data encryption module (910), compressing the encrypted data into compressed data, and transmitting the compressed data to an external storage (9000) component or to the plurality of external medical sources (930). The plurality of external sources (930) may be a hospital, a medical lab, a clinic, an emergency center, and a pharmacy. In some embodiments, the external storage (9000) may comprise a blockchain for data storage, analysis, research, and cybersecurity. Encrypted user data may be stored on the blockchain with a lifetime unique ID in order to greatly boost the information security for the user. On the blockchain, the data is stored in multiple locations to prevent insurance fraud, medical malpractice, and cyber attacks. Furthermore, the illnesses, treatments, examinations, recovery times, and prescriptions of the patient's medical history and treatment records stored on the blockchain are time stamped and can be used for R&D and big data analysis to improve the performance of all MyDoctor platforms. The external communicator module (900) may communicate with the hospital to receive medical records of the user and scheduling information for arranging an appointment, and transmits the user data and appointment requests to the hospital. The external communicator module (900) may communicate with the medical lab to receive medical records of the user, scheduling information for arranging an appointment, and lab results, and transmits the user data and appointment requests to the medical lab. The external communicator module (900) may communicate with the clinic to receive medical records of the user and scheduling information for arranging an appointment, and transmits the user data and appointment requests to the clinic. The external communicator module (900) may communicate with the emergency center to request an ambulance, and transmits the user data and a location of the user to the emergency center. The external communicator module (900) may communicate with the pharmacy to receive medical records of the user and pricing information for prescriptions, and transmits the user data, a plurality of prescriptions, and a delivery location for prescriptions to the pharmacy. The pricing information for prescriptions may comprise a pricing chart for each prescription with a list of possible locations to pick up the prescription and the corresponding prices of the prescription for each location as well as additional information (e.g. hours of operation, quantity in stock, etc.).

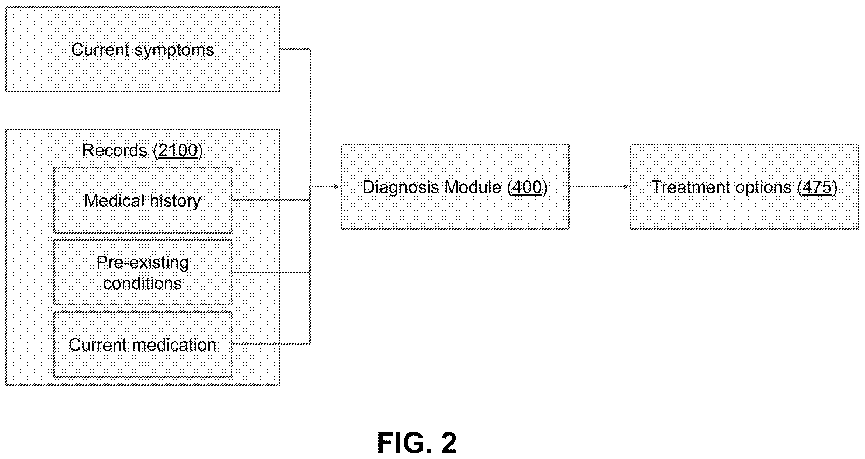

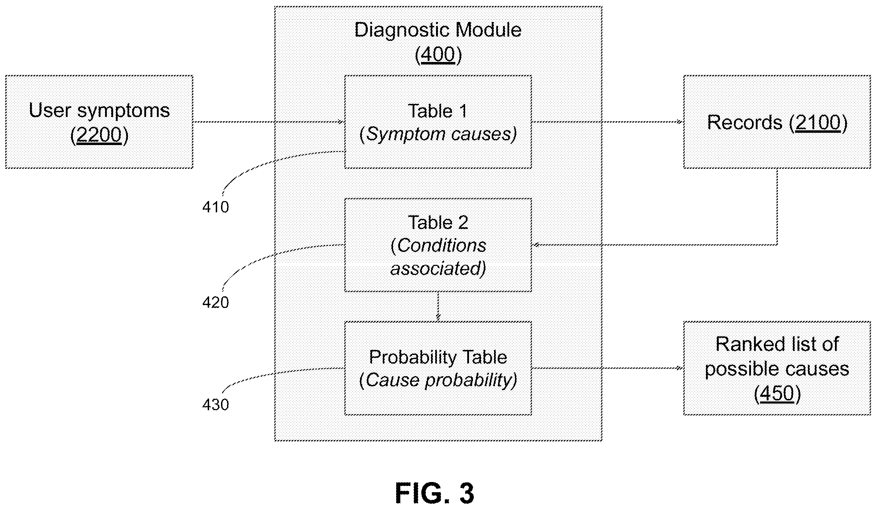

Referring now to FIGs 2-3, the present invention features a MyDoctor platform for diagnosing a medical issue of a user. In some embodiments, the platform may comprise a database of medical records (2100). The database of medical records (2100) may comprise a medical history of the user, a list of pre-existing conditions of the user, and a list of medications currently taken by the user. The platform may further comprise a diagnostic module (400) having instructions for accessing a database of disease symptoms mapped to possible causes and accepting a list of user symptoms from the user. The diagnostic module (400) may further comprise instructions for generating a first table (410) mapping each user symptom to all possible causes for the user symptom. The first table (410) may be filtered using data from the database of medical records comprising an age, a gender, a weight, and a medical history of the user. The diagnostic module (400) may further comprise instructions for generating a second table displaying all possible causes (450) that share every user symptom, accepting data from the database of medical records (2100), generating a probability table ranking all possible causes (450) based on probability of the presence of each possible cause, and determining a treatment (475) for the possible causes based on the data from the database of medical records (2100). The probability table may be generated for every user symptom. The probability weights may be calculated by dividing the number of symptoms matched between the user symptoms and the symptoms of the possible cause by the total number of user symptoms. In some embodiments, probability weights may be adjusted according to illnesses reported in a radius around the user's location. In some embodiments, the diagnostic module (400) may further comprise instructions for requesting a plurality of tests from the user to better determine the possible cause, and the MyDoctor platform (600) may further comprise testing equipment for allowing the user to carry out the plurality of tests. The testing equipment may comprise a personal vital signs monitor, a personal EKG, a glucometer, an ultrasound imaging device, a cardiac monitor, and a skin image camera. In some embodiments, the testing equipment may further comprise a blood test device and a urine test device. The equipment compatible with the MyDoctor platform (600) may be dependent on the portability and price of various testing devices and how these parameters change over time. For example, in some embodiments, the testing equipment may further comprise a portable CT scanner and a portable MRI machine. In some embodiments, the testing equipment may further comprise a LIDAR scanner capable of scanning the patient's body in a situation where there is a wound. The LIDAR scanner may be capable of measuring a depth and a severity of the wound of the patient. This information may be used to determine a treatment plan for the patient and stored in the external storage component (9000) for future treatment planning. In some embodiments, the MyDoctor platform (600) may further comprise a test analyzer module (500) for accepting test results from the user and processing the test results to aid the diagnosis platform in generating the possible cause. For a patient reporting just one symptom, the platform may check the patient's medical history, illnesses reported in the region, and perform more tests to obtain more information in order to determine the most likely illness. In some embodiments, the database of disease symptoms is stored in a cloud server and accessed by the diagnostic module (400).

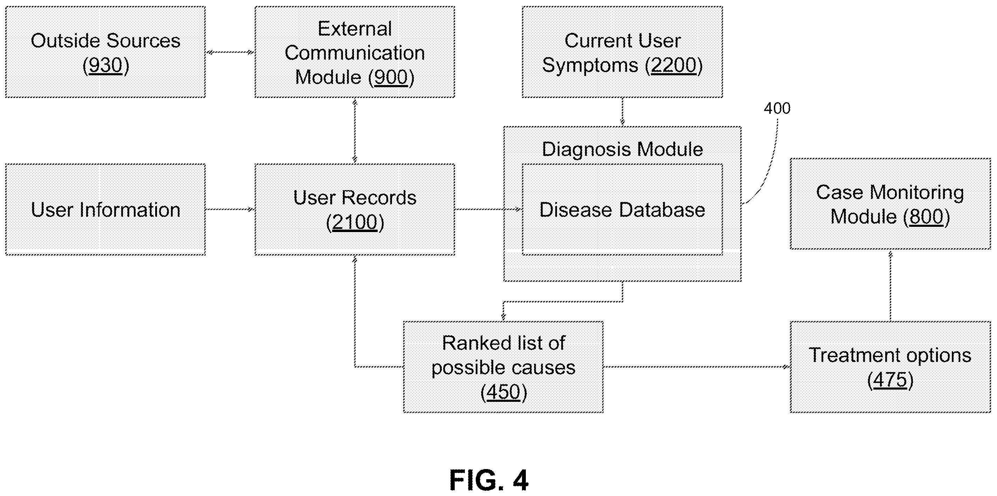

Referring now to FIG. 4, the present invention features a MyDoctor platform (600) for compiling a user's medical history from a plurality of sources and using the medical history to diagnose a medical issue of a user. The platform may comprise an external communicator module (900) for gathering information pertaining to the user's medical history from a plurality of sources. The external communicator module (900) may comprise a data encryption module (910) having instructions for accepting data from a source, and encrypting the data into a standard format. The data encryption module (900) may be capable of accepting data directly from the user, or from a plurality of external medical sources (930). The external communicator module (900) may further comprise a data compression module (920) having instructions for accepting encrypted data from the data encryption module (910), compressing the encrypted data into compressed data, and transmitting the compressed data to an external storage component (9000) or to the plurality of external medical sources (930). The platform may further comprise a database of medical records (2100). The database of medical records (2100) may comprise a medical history of the user, a list of pre-existing conditions of the user, and a list of medications currently taken by the user.

The platform may further comprise a diagnostic module (400) having instructions for accessing a database of disease symptoms mapped to possible causes and accepting a list of user symptoms from the user. The diagnostic module (400) may further comprise instructions for generating a first table (410) mapping each user symptom to all possible causes for the user symptom. The first table (410) may be filtered using data from the database of medical records comprising an age, a gender, a weight, and a medical history of the user. The diagnostic module (400) may further comprise instructions for generating a second table displaying all possible causes (450) that share every user symptom, accepting data from the database of medical records (2100), generating a probability table ranking all possible causes (450) based on probability of the presence of each possible cause, and determining a treatment (475) for the possible causes based on the data from the database of medical records (2100). The probability table may be generated for every user symptom. The probability weights may be calculated by dividing the number of symptoms matched between the user symptoms and the symptoms of the possible cause by the total number of user symptoms. In some embodiments, the diagnostic module (400) may further comprise instructions for requesting a plurality of tests from the user to better determine the possible cause, and the MyDoctor platform (600) may further comprise testing equipment for allowing the user to carry out the plurality of tests. The testing equipment may comprise a personal vital signs monitor, a personal EKG, a glucometer, an ultrasound imaging device, a cardiac monitor, and a skin image camera. In some embodiments, the MyDoctor platform (600) may further comprise a test analyzer module (500) for accepting test results from the user and processing the test results to aid the diagnosis platform in generating the possible cause. In some embodiments, the database of disease symptoms is stored in a cloud server and accessed by the diagnostic module (400).

The platform may further comprise a case monitoring module (800) having instructions for monitoring the user's health and recovery as the user carries out the treatment (475) determined by the diagnostic module (400), reminding the user to carry out a prescription related to the treatment (475), comparing an expected recovery time to an actual recovery time to determine if a secondary course of action is necessary, adjusting the prescription as the secondary course of action, and adjusting the expected recovery time based on the secondary course of action.

In some embodiments, the MyDoctor platform (600) may further comprise a treatment module (700) for generating a plurality of treatments for the plurality of possible causes generated by the diagnostic module (400) and allowing the user to select a route of treatment. In some embodiments, the external communicator module (900) may further comprise instructions for ordering the prescription to be delivered to the user. In some embodiments, the MyDoctor platform (600) may further comprise a nursing services module (1000) for allowing the user to request a registered nurse or licensed health care provider to aid the user with medical issues, testing, or treatment. In some embodiments, the MyDoctor platform (600) may further comprise a dietician (1100) module for keeping track of the user's dietary habits as data to be added to the database of medical records (2100) and for aiding the user in maintaining a healthy diet in accordance with the treatment determined by the platform. In some embodiments, the MyDoctor platform (600) may further comprise a central research module (1200) for communicating with a database of data recorded by a plurality of instances of the platform to transmit and receive test results, diagnoses, and recovery times to improve calculations and overall performance of the platform.

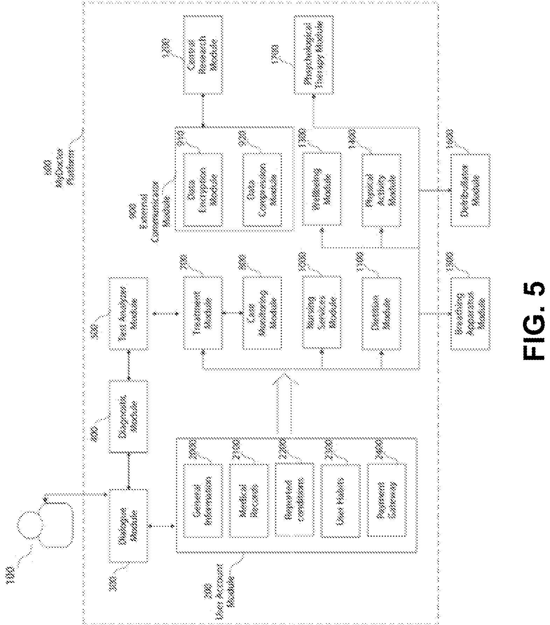

The present invention features the MyDoctor platform (600) that could be uploaded on a cloud where various modules that could be uploaded and integrated and peripherals (personal diagnostic devices) could also be connected to the platform as well. The MyDoctor platform (600) could be accessed through a smartphone application or a standalone device linked to the internet. FIG. 5 shows a diagram that demonstrates the general scheme of the invention.

The modules of the MyDoctor platform (600) may be integrated using a suitable programming language such as Python as a back-end or similar language and friendlier language such as PHP as front end. The MyDoctor platform (600) may have accessibility to communicate with various relevant parties such as medical centers including clinics, hospitals, pharmacies, Labs and emergency centers (e.g. 911). Various modules that are connected and integrated are uploaded on the MyDoctor platform (600). The MyDoctor platform (600) may be contained and executed on any computing device comprising a memory component capable of storing data and computer-executable instructions, and a processor capable of executing the said computer-executable instructions. This computing device may be a desktop computer, a laptop, a piece of computerized medical equipment, a mobile smart device, or any other computing device within the parameters listed above.

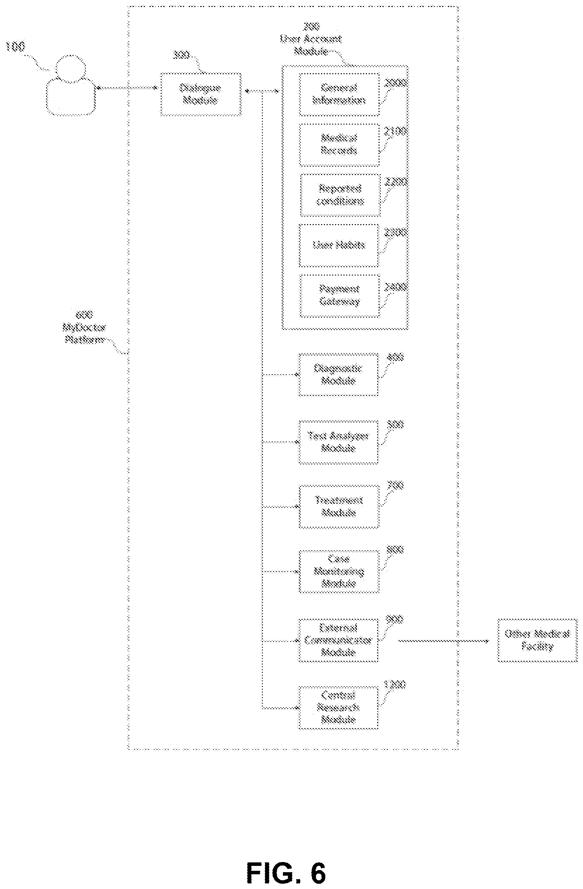

FIG. 6 shows the layout of the User Account. The User Account Module may be connected to all other modules where the data may be exchanged to and from the Patient account. Each user (100) should have an account where information about the user (100) should be entered. The required information for registration could be entered verbally or manually. If the request is verbal, the system may convert it to text using Speech Recognition algorithms (e.g. Linux Speech Recognition Software-Open Source) through the Dialogue Module (300). The user (100) should turn on the application and if it is his/her first time, a smart form has to be filled. The system may ask the user (100) questions (audibly) to fill out the form such as name, age, gender, etc. Once the smart form is filled and all mandatory questions are answered and requirements filled, the user (100) may have an active account.

Another option to expedite the registration process is to link to the National ID Number (e.g. Social Security), once the National ID is inserted the system may fill out the general information such as name, age, personal picture and fingerprint. Therefore, the user (100) may only fill out or authorize to retrieve the historical medical information and payment information for billing. In an emergency situation, an authorized personnel or emergency response team could log into the user (100) account via voice / user fingerprint / user facial recognition where this feature is important to enter into the User Account Module (200) when the user (100) is unconscious. Each time, the user (100) interacts with the system the tone of voice and facial expressions may be recorded. As the user (100) keeps using the system, the database of the tone of voice and facial expressions could be processed using Machine learning to determine the user (100) sentiments and feelings.

Voice tone analysis is critical in determining the User (100) mood, stress, pain, anxiety, and depression. As more data is collected on the user (100), the MyDoctor Platform (600) can immediately determine the urgency of the user (100) request. The User Account Module (200) may consist of many features and options including:
1. General Information (2000)
2. Medical Records (2100)
3. Reported conditions (2200)
4. User Habits (2300)
5. Payment Gateway (2400)

As part of the User Account Module (200), the General Information (2000) of the user (100) may contain information to identify the user (100). This information could also be shared with other medical providers to identify the user (100) and to obtain the user (100) medical records.

Medical records (2100) from the user (100) may be retained to provide better and expedited diagnosis and to assist with the Central Research Module (1200). At the same time, the User (100) external medical records can be uploaded to the user medical records (2100) through connecting the Electronic Health Record (EHR) or Hospital Information Support System (HISS) to the MyDoctor platform (600). The Medical Records (2100) may be updated for any cases of illness, stress, discomfort, allergies to track the health of the user (100) with time. The Medical Record (2100) may communicate with Case Monitoring Module (800) so that the progress of the treatment can be recorded. Tests Analyzer Module (500) is also linked to the Medical Record to log the results of tests and examinations. External Communicator Module (900) is linked to the Medical Record to feed the Medical Records from the external facilities such as labs and physician input.

At the same time, the External Communicator Module (900) may provide outside medical facilities such as physicians with access to the patient's medical records (2100). The Medical Records may also communicate to the Diagnostic Module (400) to support the diagnosis process and to the Treatment Module (700) to record the prescribed treatment. It is also linked through a one-way communication channel to feed Central Research Module (1200).

For underage (e.g. infants) or those who do not have the capacity to use the MyDoctor Platform (600), for any reasons, a trustee could interact with the MyDoctor Platform (600) on behalf of the user (100).

With more interaction and usage from the user (100), the Reported conditions (2200) of the user (100) may be kept and shared with the Central Research Module (1200). This information is critical in M.L. in predicting illnesses and to track the user (100) health as they get older. This information is useful to share with medical insurance companies to determine high risk patients, high risk age and to issue preventive care before a serious illness might occur.

The User Habits (2300) are stored and shared with other modules in the MyDoctor Platform (600). This information is used with the Dietitian Module (1100), Central Research Module (1200), Wellbeing Module (1300), Physical Activity Module (1400) and Psychological Therapy Module (1700). The information collected could be daily exercise, nutrition, favorite foods, sleep habits and quality of sleep. The Payment Gateway (2400) is used to work with the External Communicator Module (900) to make payments to Pharmacies, doctor's offices, and to purchase add-ons to Modules or subscription services.

The Payment Gateway (2400) may have two options, meeting all PCI DSS requirements for the user (100) to make payment as payment is needed at the time of service. Or to have the User (100) payment information on file during the creation of the User Account, where PCI DSS requirements may be met at the back office.

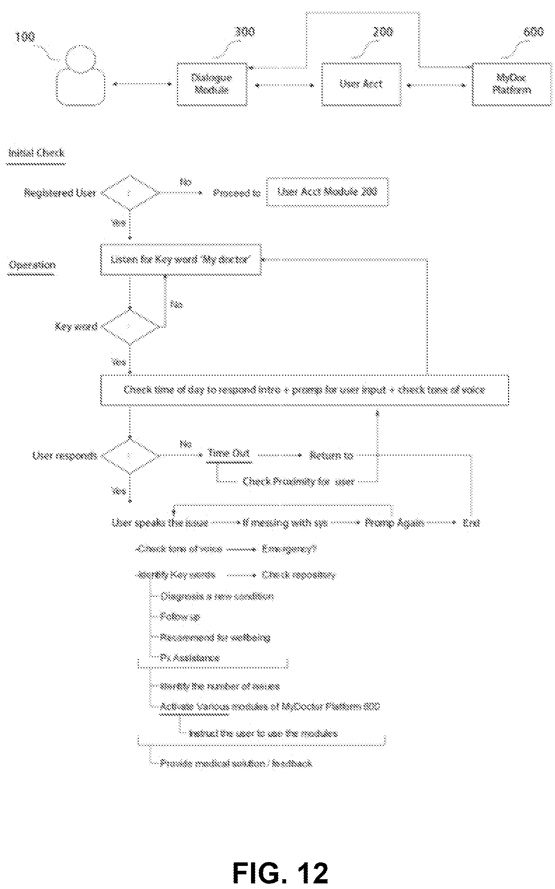

FIG. 12 shows the layout of the Dialogue Module 300. The Dialogue Module (300) may be connected to other modules where the data may be exchanged to and from the user (100) and the logic flow process. This module may be the interface between the user (100) though the User Account (200) and the MyDoctor platform (600). The objective of this module is to convert the text to verbal conversion and vice versa. This module may employ the speech recognition and Natural Language Processing to communicate with the User (100). The system may have predefined phrases with multiple versions for the same phrase. For example, whenever the User (100) calls the system "MyDoctor" the system may reply and ask the user (100) "how can I serve you". The user (100) could simply say anything. However, the system may focus on medical conditions using algorithms such as BERT as a tool to perform NLP. The dialogue between the system and the user (100) may take a format of question and answer. When the user 100 says something relevant to his physical or even psychological condition the system may interact and ask questions to diagnose the condition as a first step. To demonstrate the possible dialogue, the following scenario could take place:

The System: "Good Morning Mr. David, I am your physician Dr. Mazen; how can I help you?" (The greetings may depend on the time of the day. Other welcoming statements could be prompted to give the system more natural feelings. In case, the user (100) is following up with the system after reporting a medical condition the greetings statement may be different. It could be "how do you feel now? A predefined protocol may be loaded to manage the flow of the communication)

The user (100) has to answer the system. The following scenarios could take place:
- The User (100) keeps silent
- The User (100) could keep talking in a subject irrelevant to his/her physical or psychological condition
- The User (100) states his/her problem but it could be not in a clear manner.

For each scenario a certain course of action should take place. The following is the typical system's behavior for each one of the above scenarios:
1. If the user (100) keeps silent the system after 10 seconds may rephrase the greetings and the question. If the user (100) keeps silent the system may be on hold mode. This behavior could be different in case depending on the situation. Adding a sensor (infrared or camera) could also help the system to know if the User (100) is within the vicinity of the device. This may be doable if the standalone version of the proposed invention is adopted.
2. If the user (100) starts saying nonsense words the (100) system may state that "I am here to provide you with a medical care service. I can only help you if you need medical help". The system may utilize cameras and other sensors to determine if the user (100) is in distress and call for an emergency response team as necessary. If the user (100) keeps playing with the system the system may timeout with "Sorry, I cannot help you."
3. If the user (100) says anything relevant to a medical issue the system may recognize the words of attention (feel/headache/medicine etc.) which could be one out of many defined in the repository dictionaries with synonyms.

The system may lead the conversation based on the initial input of the user (100). The conversation between the user (100) and the system may search a predefined repository to determine what task to perform and to focus on one of the following tasks:
- Diagnosis a new condition
- Follow up of existing condition
- Psychological Therapy
- Recommendation for wellbeing
- Medical assistance such as ordering medicine from pharmacy or making appointment with specialist

By using NLP this module may specify the task and relevant modules may be triggered with its appropriate flow of the dialogue. If the user (100) mixes up more than one topic. The system may segregate the issues and address each topic separately. For example, if the User (100) says "I have a headache and I need to order a refill for my diabetic medicine." In this case, the system may address the headache separately from ordering the refill but before it does so the system may verify that there is no association between both requests. Structuring the conversation by the system is important to achieve a certain task.

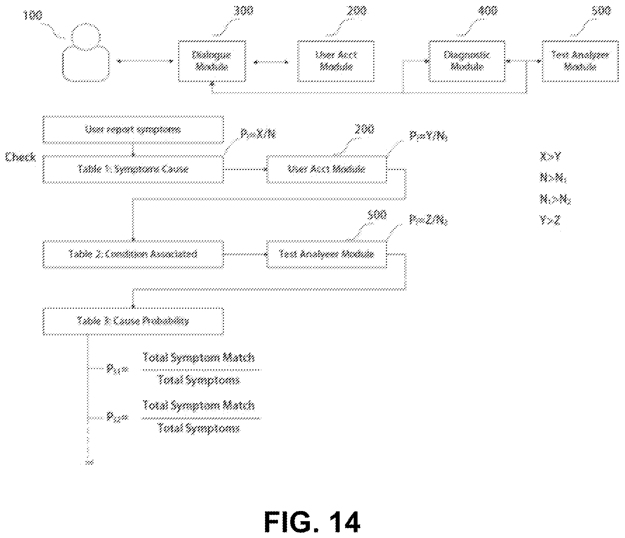

FIG. 14 shows the layout of the Diagnostic Module (400). The Diagnostic Module (400) is a repository of all human being known diseases with their signs and symptoms and the tests that are necessary to confirm the condition along with algorithms employing the deep learning and Artificial Neural Network concept.

The Diagnostic Module (400) is communicating with Dialogue Module (300) to receive the symptoms from the patient and to provide the patient with the necessary tests and examinations to be performed. Medical Record Modules also communicates with the Diagnostic Module (400) as input to the search algorithm. Test Analyzer Modules (500) Module provides the results of the various tests to be used in the diagnosis process. This module may be triggered once the task requested by the User (100) is determined in the Dialogue Module (300) to be a request to diagnose the User (100) medical conditions. The key words and symptoms that the user (100) mentions to the Dialogue Module (300) may be used to search in the Diagnosis tables. The Module may ask the user questions regarding the symptoms. The more variables (symptoms) provided the better search could be conducted. The input from the vital signs device may also be used as a search variable. To be able to conduct the diagnosis, certain input may be essential to isolate the condition. To narrow the search, three tables may be searched initially. The first table is symptoms cause table where all symptoms are tabulated and under each symptom all possible related causes (diseases) are tabulated. The list of the symptoms (number of columns) could be more than 84 symptoms. The symptom could be noun (Fever) or adverb (e.g. cannot breathe). The system may conduct its initial filtering. For example, if the reported symptom is only fever, the system may focus on all possible causes for fever and run a sanity check for each possible cause. Some of the causes could be a group of diseases such as infection, dehydration, medications currently taken or heatstroke. In this case, another table is built for this group. The criteria for sanity check are basically age, gender, weight, severity of the symptoms and user (100) medical records (2100). For each cause (disease) there are factors that may affect the probability of the cause. Initially all possible causes may have equal probability so if there are four possible causes the probability for each one of them is 25%. When the system conducts the filtering for each cause certain predefined factors may affect the probability.

For example, if the patient is not an infant the teething as a cause for fever may have 0% probability. Teething as a possible cause has been excluded with the assumption that the User (100) is adult based on the general information. Similarly, if the season is winter time the probability of heat stroke is low and could be 0%. By this the system may narrow the initial search.

The second table is condition associated symptoms. For example, if the patient only reported having a fever as a symptom, all possible causes may be tabulated. The second table may show all causes for fever and the symptoms of each condition associated with fever. It can be observed that headache is a common symptom between two causes namely: heat stroke and inflammation. Therefore, the system might test these two possible causes by asking the user (100) if they have a headache. If the response is negative this may normalize the weight of inflammation and heat stroke and if the response was positive this should add a higher weight to the probability of heat stock and inflammation over other possible causes (infection).

The third table is the cause probability weight table where the symptoms of each possible cause are ranked based on the probability since we cannot assume that all symptoms bear the same weight. In some cases, there may be a predefined probability weight for each symptom. For example, each symptom of infection has its own weight. Some symptoms are primary and should have a higher probability weight such as fever while secondary symptoms such as digestive upset should bear lower weight. The weight of the symptoms could also be influenced by various factors. Age, gender, severity of the symptom, the duration of the symptoms and user (100) medical records (2100) and history may affect the weight to filer the symptoms cause table, similarly if the weight of the patient is reasonable obesity as a cause for sweating is going to have 0% probability. The symptoms probability weight table may be created for each reported symptom.

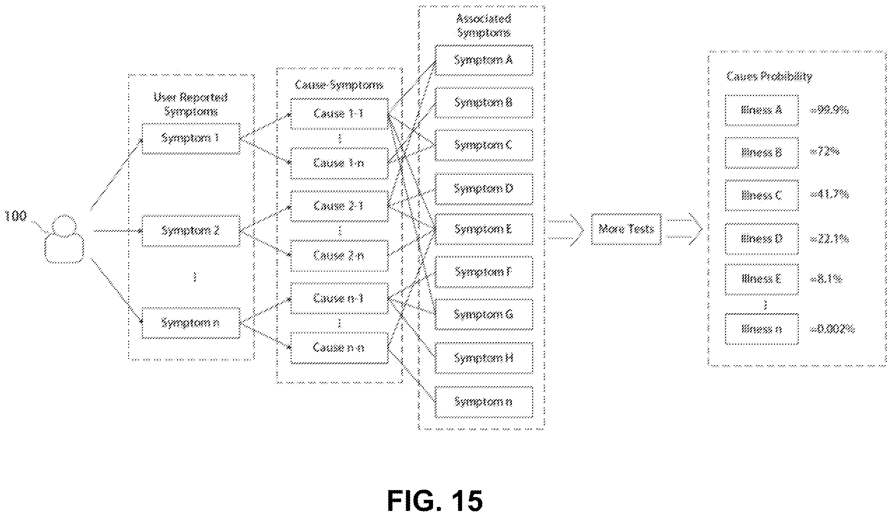

FIG. 15 illustrates the above filtering and probability analysis to narrow the possibilities and determine the next step in the diagnostic process. Based on the probability analysis the system may either ask the patient more questions to narrow the possibilities furthermore or the system might request from the patient to do some tests if the patient couldn't provide additional symptoms. Some symptoms are purely descriptive such as pain or sweating which could be severe or mild (descriptive) while others could be measured such as fever (discreet). For those that could be measured the system may request the patient to do the appropriate exams. The input of the exams may be used to narrow the diagnostic tables furthermore. The medical exams could be certain lab exams or x-rays. The input of those exams should be fed to the system in the patient record. The Diagnostic Module (400) may read the input from the User (100) medical records (2100) to conduct the diagnosis. It is possible that User (100) is requested to perform additional tests to enhance the probability and diagnostic. The Random Forest Classifier could perform the diagnosis which is one of the functions under R programming language. In addition, the Diagnostic Module (400) may have a table of all tests and procedures to be used to verify any possible condition (disease). The table lists all tests and procedures and for what condition should it be requested. For example, if the patient has some or all of the following symptoms:
- Increased thirst
- Frequent urination
- Increased hunger
- Unintended weight loss
- Fatigue
- Blurred vision
- Slow-healing sores
- Frequent infections
- Areas of darkened skin, usually in the armpits and neck

The Diagnostic Module (400) may flag the User (100) with high probability of being a diabetic based on running the symptoms by the diagnostic tables. In order to verify and classify what type of diabetes and how bad the patient's condition is, the system may request from the User (100) to use Glucometer from the patient's kit to measure the sugar level randomly. In addition, the system may ask the patient to perform a fasting blood sugar test using the Glucometer and may be Oral glucose tolerance test in the lab if the patient is a pregnant woman for example. Those tests may be determined based on the test and procedures table. The results of the tests and procedures may be read and assessed by the Diagnostic Module (400). Test evaluation table under the Test Analyzer Module may provide interpretation to the result of the medical test.

The Diagnostic Module (400) may have the capability to conduct visual diagnosis. For example, if the patient complains about a red spot. The system may ask the patient to take a picture using a high-resolution camera and the system may use Machine Learning to determine the possible condition. If the system couldn't provide a diagnosis with more than 99% probability. The system may request the patient to see a physician and the system may provide assistance in identifying the appropriate physician and making appointments with him.

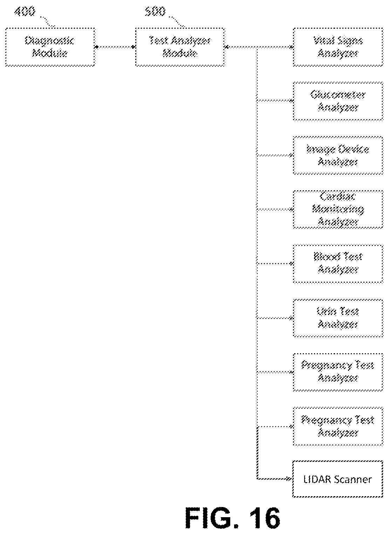

FIG. 16 shows the Test Analyzer Module, where the Diagnostic Module (400) may determine the required test and the Test Analyzer Module (500) may conduct the analysis based on predefined criteria. The Test Analyzer Module (500) may read the input from various peripherals including Personal Vital Signs, Personal EKG, Glucometer, Ultrasound Image Device, Cardiac Monitoring and Skin/Eye Image Camera and additional handheld medical devices. The readings from those devices could be via Bluetooth, zigbee, WiFi, and NFC. It also reads the external tests including lab test results and other forms of tests such as MIR images either through email, direct link or entered by the patient either verbally or in writing. The analysis of the test results shall take place in this module, where all the information from different medical equipment are converted into the same format and fed to the Diagnostic Module (400). The results of the analysis could be fed to the Medical Record Module (2100) and to the Central Research Module (1200). The Test Analyzer Module (500) has multiple functionalities and each function could conduct certain analysis. The following are some functionalities that may be fed to the Test Analyzer Module (500):
i. Vital Signs Analyzer: This function processes the readings of the vital signs and correlates the vital signs readings with other variables to assess the patient condition and feed the results to the Diagnostic Module (400). The readings of the vital signs may be communicated to the Medical Record Module to log the input with the date and the time.
ii. Glucometer Analyzer: this function processes the readings of the blood surge and feeds the results to the Diagnostic Module (400). The various readings of the blood surge are recorded with time where analysis could be conducted. Accordingly, the Diagnostic Module (400) could adjust the treatment plan or the doses of insulin for example.
iii. Image Device Analyzer: this function may employ Machine Learning and image recognition to determine the results of reading for various types of images including ultrasound images, skin images, x-ray images and eye images.
iv. Cardiac Monitoring Analyzer: this function may use an algorithm to perform the necessary analysis for cardiac patterns. With more usage from the user (100), more information is gathered and any abnormalities can be detected at an earlier stage.
v. Blood Test Analyzer: this function may determine the normal range and relevant analysis based on the results of various blood tests.
vi. Unrein Test Analyzer: this function may determine any abnormality in the tests and conduct the analysis of the various unrein tests.
vii. Pregnancy Test Analyzer: this function may monitor the menstrual period of a female user (100), to determine the ovulation date for the highest chance of pregnancy and to detect early pregnancy to notify the female user (100).

In addition to above in general all tests relevant to cellular and chemicals tests, diagnostic imaging, genetic testing, physical and visual examination and measurements (e.g. lumbar puncture) could be analyzed under Test Analyzer Module 500. Additional functionalities could be added as technology improves and reduces the size of analyzers to be more portable.

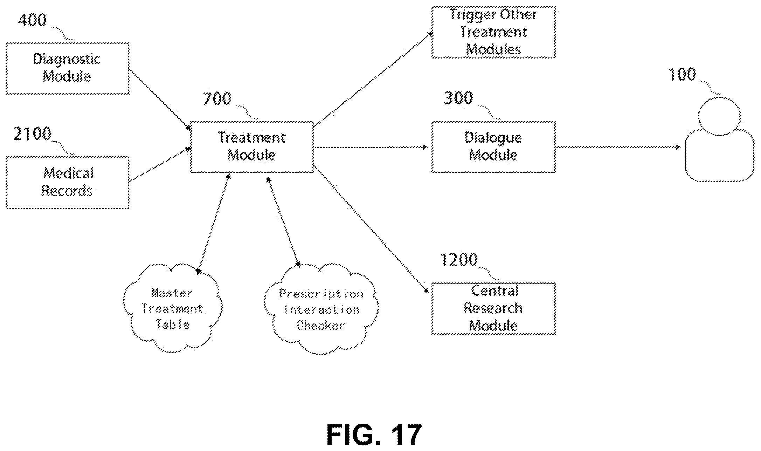

FIG. 17 shows the Treatment Module (700). The Treatment Module (700) takes the input from the Diagnostic Module (400) and Medical Records (2100) to determine the suitable treatment and prescriptions for the user (100). For example, the Medical Records (2100) could show that the user (100) has an allergy for certain prescriptions. The Treatment Module 700 in such cases could find an alternative medicine. Once the medical condition is determined by the Diagnostic Module (400) with high probability, the Treatment Module (700) may determine the appropriate treatment. For each condition (disease), there may be a predefined treatment plan and recommendation in a repository.

The treatment plan for each condition should be adjusted to suit the patient and to consider all relevant factors related to the patient such as age, medical history and other diseases. The dose of the medicine may also be calculated based on those factors. For example, if the Diagnostic Module (400) performed tests and determined the user (100) condition is type 2 diabetes, the Treatment Module (700) may provide the patient with the proper treatment plan. The treatment repository may provide how to manage type 2 diabetes with the following factors to be considered:
- Weight Control
- Healthy eating
- Regular exercise
- Possibly, diabetes medication or insulin therapy
- Blood sugar monitoring

The above treatment / recommendations may reside in the repository under the treatment of type 2 diabetes table. For each of the above variables a table with more details may be available. For example, weight control and healthy eating as a treatment recommendation are common in many diseases. Therefore, weight control and healthy eating may be treated as a standalone variable. At the same time, weight control should be defined and similarly healthy eating. Therefore, for weight control as a variable there may be a table to determine what is the perfect weight based on relevant factors such as height, gender, body mass index (BMI) and age.

For healthy eating, the Treatment Module (700) may trigger the Dietitian Module (1100) and similarly for additional exercise and physical therapy the Treatment Module (700) may instruct the user (100) to the Physical Activity Module (1400). The Treatment Module (700) may instruct the user (100) with the frequency of checking the blood sugar and the system may monitor the blood sugar and maintain the readings. The Treatment Module (700) may also prescribe the suitable medication based on the user (100) condition. Other recommendations based on the user (100) condition may also be extended. For example, if the patient's body mass index (BMI) is greater than 35, the Treatment Module (700) may recommend weight-loss surgery (bariatric surgery).

The Treatment Module (700) may not just provide the recommendation but may develop a treatment plan suitable to the patient. For example, if the user (100) weight is average, the Treatment Module (700) may not have it as part of the treatment plan. However, the Treatment Module (700) may direct the User (100) to the Dietitian Module (1100) to assess whether the user (100) may follow healthy eating habits and may provide examples and recommendations of what can improve the healthy eating habits. The Master Treatment table is the repository for all treatments. For each condition (illness), there is a treatment plan which includes general recommendations, options, medicine and alternative medicine. The treatment plan for each condition may be adjusted to suit the user (100).

The general recommendations may be provided to the patient through Dialogue Module (300) as a conversation. For each variable more details may be retained to provide the patient with details. The medicine may be generic in the Master Treatment Table. Also, there could be many medicines for the same condition (illness). The Treatment Module (700) should choose the most suitable medicine based on factors relevant to the user (100). When there are multiple medicines the primary medicine may be flagged and prescribed. For example, if the condition is hypertension Thizide Diuretics may be prescribed since it has less side effects. The Treatment Module (700) can trigger the Case Monitoring Module (800) for some conditions to determine the effectiveness of the prescription. In case the prescription is not effective the Case Monitoring Module (800) may request the Treatment Module (700) for alternative solutions.

There may be a table of brands of medicine. For each brand, there may be a dosage calculator. The calculator may determine the dosage including the frequency and duration of using the medicine based on relevant factors such as age, weight and severity of the condition. The age and the weight of the patient may be imported from the User Account Module (200) and the severity of the condition may be imported from the Diagnostic Module (400). The side effect and direction of the prescription may be available to be shared with the user (100) before taking the prescription.

The Treatment Module (700) may initialize a drug interaction checker to check the user (100) other prescriptions, medical records (2100) or special conditions such as pregnancy to the conflicting medicine field to flag the conflicting prescription or a condition that could prevent taking the prescription. If the conflicting prescription is flagged, the Module may search for alternatives either for the condition under treatment or other prescriptions. Price of the prescription may also be available and the Treatment Module (700) may always suggest the cheapest brand as the first option and account for the user (100) insurance coverage, when the side effects and the other factors of the various brands are the same.

The availability field may determine where the medicine can be collected. The Treatment Module (700) may give the patient the option to order the prescription directly from the pharmacy to be delivered or to be picked up. The availability field may provide all pharmacies within the vicinity of the patient where the prescription is available. The request of the prescription to the pharmacy may be processed through the External Communication Module (900). The rating field may collect the effectiveness of the prescription based on other patients input and the results collected from the Case Monitoring Module (800). The rating filed could help in conducting research and it may be linked to the Central Research Module (1200).

FIG. 17 illustrates the Case Monitoring Module (800). Once a User (100) is diagnosed and begins the treatment. The MyDoctor Platform (600) may monitor the user (100) recovery and health through this module. Each treatment has a treatment cycle before the user (100) is fully recovered. At the same time, the user (100) could encounter some complications or the prescribed treatment could be ineffective. The MyDoctor Platform (600) may monitor the user (100) throughout the recovery process and for each treatment, the expected progress of the treatment may be tabulated in the Medical Records (2100).

For example, if the diagnosis of the condition was an infection and the MyDoctor Platform (600) made a prescription for certain antibiotics, the Case Monitoring Module (800) may compare the expected recovery time to the actual recovery time. If the recovery did not occur as expected the Case Monitoring Module (800) may provide a secondary course of action, which could include changing the antibiotics or conducting more tests with the Diagnostic Module (400) and the Test Analyzer Module (500).

The input for the Case Monitoring Module (800) may be based on the user (100) inputs and the results from the Test Analyzer Module (500). The Case Monitoring Module (800) could even request additional diagnostic measures though the Diagnostic Module (400). The Case Monitoring Module (800) may continue monitoring the condition until the user 100 is fully recovered. In some cases, the condition could be chronic such as hypertension and the Case Monitoring Module (800) may monitor the condition and provide the user (100) with recommendations and make adjustments of the prescription. The data collected from all users (100) of the Case Monitoring Module (800) could provide a significant improvement in the medical field and the data collected may be stored in the Central Research Module (1200).

The Case Monitoring Module (800) may import the prescription information prescribed by the Treatment Module (700) and may provide a reminder to the user (100) on when to take the prescription. Also, the user (100) may be reminded to refill the prescription prior to expiration or exhaustion of the prescription. The reminder could be in various forms including verbal message, audible alerts, or text messages. The Case Monitoring Module (800) may have a Master Table of all the treatments and the expected progress of the results in order to assess the effectiveness of the treatment. For example, if the Diagnostic Module (400) determined the condition as a hypertension and the Treatment Module (700) prescribed Thiazide diuretics as the medicine for treatment and if the condition based on the readings of the pressure over a period of one week after taking Thiazide diuretics continues not to be stable. The Case Monitoring Module (800) may trigger the Treatment Module (700) to find another alternative treatment.

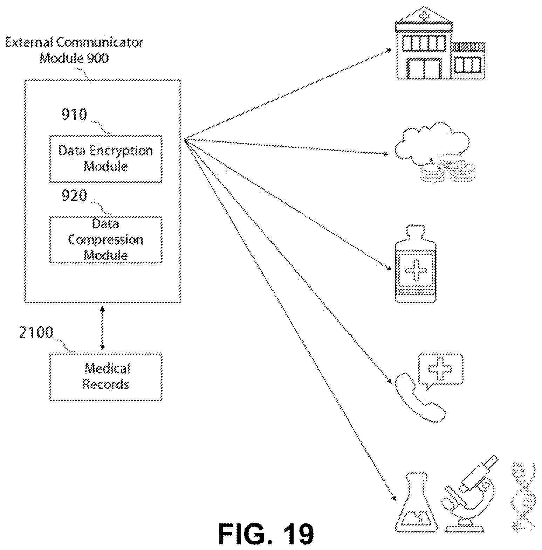

FIG. 19 shows the External Communicator Module (900). The External Communicator Module (900) links the MyDoctor platform (600) with all external parties including clinics, hospitals, emergency centers (ambulances) and pharmacies. The External Communicator Module (900) also consists of a Data Encryption Module (910) and Data Compression Module (920). The Data Encryption Module (910) translates all data in different formats to a standardized usable format when receiving information from hospitals, labs, clinics, emergency centers and pharmacies. At the same time The Data Encryption Module (910) converts outgoing data to a format that is readable and usable for the hospitals, labs, clinics, emergency centers and pharmacies. Before any data is transferred out, the Data Encryption Module (910) encrypts all data collected from the User (100) in accordance to industry standards, or blockchain and then compresses the encrypted data utilizing the Data Compression Module (920) in accordance to medical industry standard compression like Diacom.

In order for the solution to work effectively, external parties including hospitals, pharmacies, labs, and emergency centers and nursing service providers should register with the system to receive requests and share data. Different tasks should be able to be executed and data to be shared by each party. Pharmacies should be able to share pricing information including sale items with the External Communicator Module (900) and the Payment Gateway (2400) may be able to communicate with each of the external parties to make payment when payments are due. Hospitals scheduling systems should be compatible with the solution so the appointment could be booked automatically or in a format that can be translated by the Data Encryption Module (910). The medical records (2100) of the hospital should also be able to feed the system and vice versa.

Pharmacies should be able to receive prescriptions developed by the Treatment Module (700) with instructions such as location for the delivery of the prescription. In line with this, the MyDoctor platform (600) may be capable of listing out all pharmacies in a vicinity of a location (i.e. a location of the user) capable of providing the prescription. Labs scheduling systems should also be connected to the solution so an appointment can be scheduled. The test and procedures determined by the Diagnostic Module (400) should be delivered to the lab. At the same time, the lab either uploads the results or the Lab system could be accessible by the Test Analyzer Module (500). Emergency Centers should be able to receive a request for an ambulance with a description of the emergency and the user (100) location should be sent automatically from the smart phone or the standalone device. In a simplistic format and before the adoption of the solution by the various medical facilities, the system should be able to conduct an auto call to the emergency center (e.g. 911) and request a help through voice message with the location. Such emergency calls should be triggered either by the user (100) or as a result of vital signs readings when the user (100) is susceptible to emergency for example heart attack.

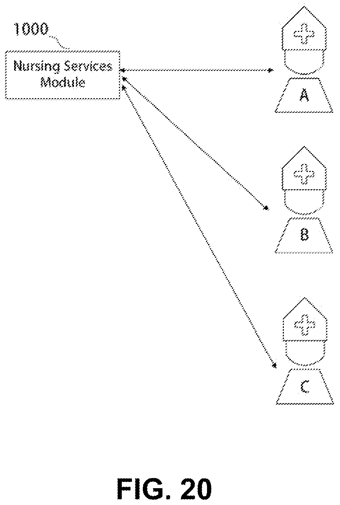

FIG. 20 shows the Nursing Services Module (1000). The Nursing Services Module (1000) may work with registered nurses or licensed health care providers to sign in and be available to be hired by the user (100) as on-demand service. Volunteers could also register to render their services to the user (100) in need and receive a log of service hours provided, where the volunteer can declare this as charity work, pro bono, or community service. The user 100 could look up available nurses or volunteers in his/her neighborhood or could initiate an ad with his/her needs and the nurse or volunteer who is interested could respond and apply to offer their services.

Those who are interested to offer their medical services, whether they are volunteers or registered nurses should register via an application or through a web page. Basic information should be entered with a brief biography as well as their photo, licenses and resume. The registered nurse or volunteer may go through a background check. The background check could take various scenarios such as a recommendation from an institution or security office. The background check is important for the security of the user (100) and to ensure the minimum necessary experience as a service provider. Once the registration is completed, the nurse or the volunteer could trigger his or her availability along with his or her location of coverage. When the user (100) requests nursing services, the application may display all available service providers in the neighborhood. The nurses could determine their hourly rates while volunteers could provide the number of hours that they are willing to allocate. Upon selection of the nurse by the user (100) a message may be sent to the nurse with brief information about the patient condition, history, the patient location and the sought services. The nurse could decline or accept the assignment. Then the nurse or service provider may be dispatched to the user (100) location and after the service, the user (100) can rate the service provider where the rating may be shared to other users.

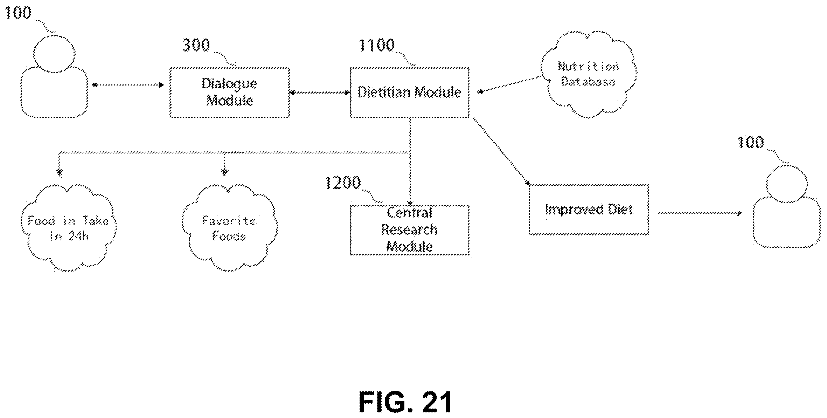

FIG. 21 shows the Dietitian Module (1100). The Dietitian Module (1100) may play the role of the dietitian and may put a plan that fits health goals, food preferences and lifestyle. For example, the Dietitian Module (1100) could monitor user (100) carbohydrate intake and determine the amount of carbohydrates she/she needs to intake with each meal and snacks to keep his/her blood sugar levels more stable.

The Dietitian Module (1100) may conduct an initial assessment of the user (100) eating habits along with a check on the Medical Records (2100). The dialogue that may take place between the user (100) and the Dietitian Module (1100) is to complete an eating habits assessment form. A questionnaire may be filled based on the input of the user (100). The purpose of the questionnaire is to determine the food that the patient eats, the quantities and preferences. For example, the Dietitian Module (1100) may ask the user (100) the following questions:
- How many meals do you have every day?
- Usually what do you have in each meal?
- What did you have yesterday?

For some responses, the Dietitian Module (1100) may request the quantities if the quantities are not given. Also, the quantities could be in a descriptive form. For example, if the user (100) mentioned that he eats cashew nuts, the Dietitian Module (1100) may help the user (100) to provide a rough estimate of the quantities by accepting answers like a handful, or a few bites. The response of the user (100) may be entered into two tables. One is the user (100) food of the last 24 hours (an example is displayed as Table 6) and typical food that the user 100 usually eats. Many individuals have non-routine food they eat. The Dietitian Module (1100) may extract such information and tabulate them for analysis.

The Dietitian Module (1100) may have a table of the ingredients and calories of all foods. Based on the user (100) input the Dietitian Module (1100) may convert the food into calories and ingredients. For example, if the patient eats cashew nuts regularly and if the average of the user (100) intake is around 100 mg the Dietitian Module (1100) may convert the 100 gm of cashews to its ingredient. When the user (100) enters a recipe such as pasta, the system may have a table with the breakdown of the recipe. If the dish is not predefined, the Module may ask the user (100) to provide the ingredients of the dish. Based on the analysis of the ingredient intake the Dietitian Module (1100) may identify the elements such as a certain vitamin that the patient does not take sufficient quantities and may make recommendations of foods that contain the missing elements.

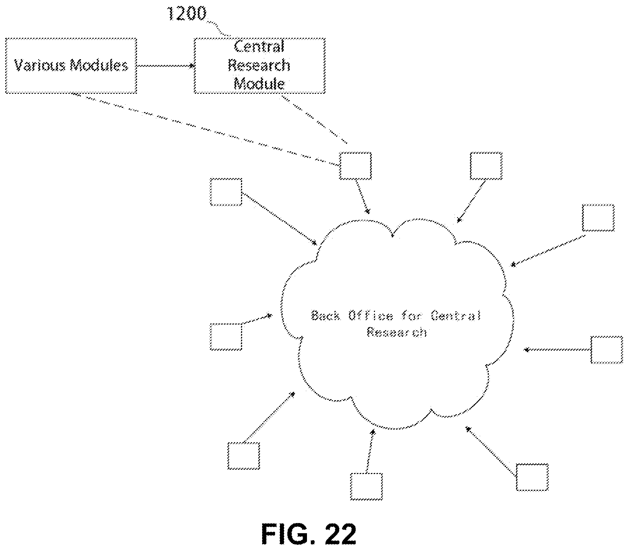

FIG. 22 shows the Central Research Module (1200). The Central Research Module (1200) operates in the back office, where data is collected for the purpose of medical research. Mass data collected from all the MyDoctor Platform (600) users, the effectiveness of medicines from Case Monitoring Module (800) could be improved. By analyzing the correlation between the tests and the diagnoses for the user (100) the unnecessary tests could be eliminated. the illness and treatment of each user (100) is analyzed. The collected data may be used to segregate users (100) by ethnicity and DNA to analyze the probability of recurring illnesses and by utilizing data science along with Machine Learning (M.L.) to provide preventive medicine. The data could be available as open source for researchers globally.

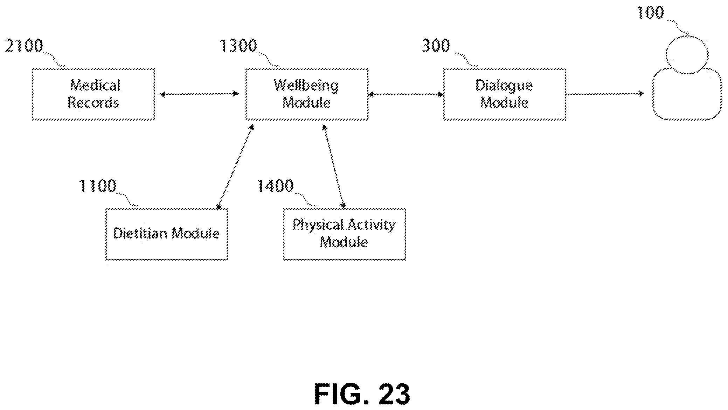

FIG. 23 shows the Wellbeing Module (1300). The Wellbeing Module (1300) may provide general recommendations that could improve user (100) wellbeing, working in cooperation with the Dietitian Module (1100) and the Physical Activity Module (1400) to recommend certain supplements that are determined based on the user (100) special needs taking into consideration of age, physical condition and the Medical Records (2100) of the user (100) including any genetically inherited diseases. In some embodiments, the Wellbeing Module (1300) may apply data science to specify relevant screening tests, wherein such exercise would be challenging to be carried out manually by a human being. The said relevant screening tests may help to proactively detect any potential disease at an early stage with the screening tests based on a plurality of factors such as age, weight, patient medical history, patient family history, etc.

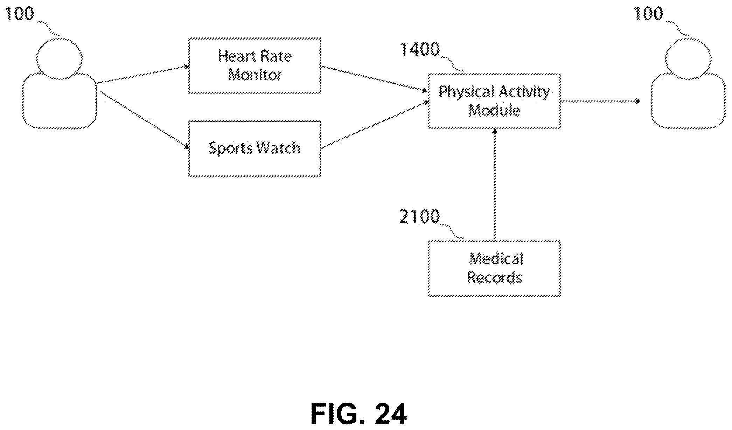

FIG. 24 shows the Physical Activity Module (1400). The Physical Activity Module 1400 may provide physical activity tracking and instructions to the user (100). The Physical Activity Module (1400) can track and collect data on the amount of exercise the user (100) performed in a day and provide feedback on the result to instruct the user (100) to make improvements to obtain the result the user (100) is seeking.

The Physical Activity Module (1400) can also assist the user (100) with physical therapy and improve the user (100) range of motion through instructional videos and monitoring the user (100) vital signs.

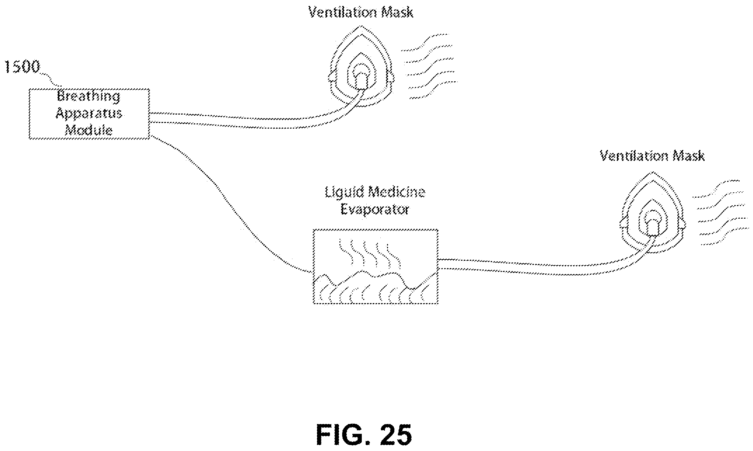

FIG. 25 shows the Breathing Apparatus Module (1500). The Breathing Apparatus Module (1500) can function as a ventilator that moves breathable air into a non-invasive ventilation mask or bag valve mask to deliver breaths to the user (100) who is physically unable to breathe or is breathing insufficiently. The Breathing Apparatus Module (1500) can also function as a nebulizer to assist users (100) that have asthma by delivering liquid medicine via mist through the ventilation mask. The Breathing Apparatus Module (1500) can be set up initially as a long-term breathing support system for the user (100) from the beginning or can be activated by the user (100) if the user (100) requests symptoms that require breathing support.

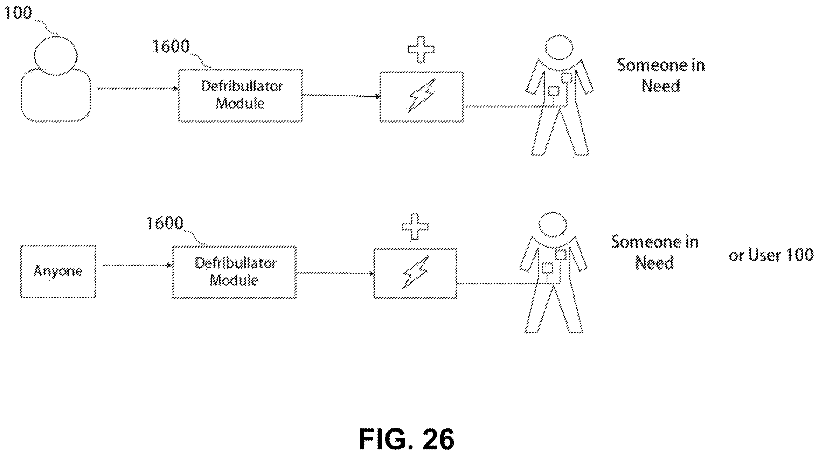

FIG. 26 shows the Defibrillator Module (1600). The Defibrillator Module (1600) is a treatment for life-threatening cardiac dysrhythmias by delivering a dose of electric current to the heart. This module can be used as an emergency device by any user to help someone in need. Or can be utilized by someone to assist the user (100) who might be in need of emergency medical assistance. In an emergency situation, the MyDoctor platform (600) can instruct the User (100) or a person who can provide the emergency care through the Dialogue Module (300) to utilize the Defibrillator Module (1600). Turning the MyDoctor Platform (600) as an emergency device to save other people. When the platform based on the readings of the vital signs determines the condition of the patient as "code blue" the MyDoctor platform (600) may activate a siren in addition to activating a call to the emergency center, and the MyDoctor platform (600) may instruct the User (100) or a person who can provide the emergency care on how to use the defibrillator to perform emergency medical service to the person in need or to the user (100).

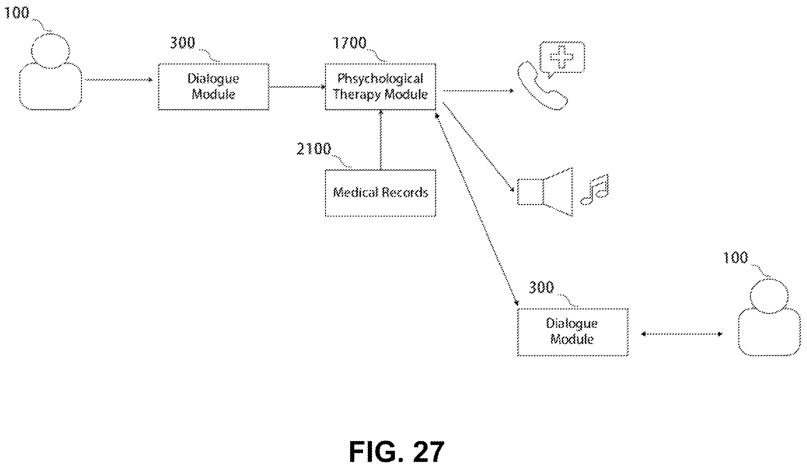

FIG. 27 shows the Psychological Therapy Module (1700). The Psychological Therapy Module (1700) may provide diagnostic and psychological assessment to User (100) to determine the condition and extend recommendation. The facial sentiment and voice tone could be used to assess the progress of the condition. Utilizing the Dialogue Module (300) the tone of the User (100) voice is analyzed to determine if the User (100) is in distress, pain, anxious, depressed or angry. The MyDoctor Platform (600) may utilize machine learning to adapt to the User (100), with a base built from the User Account Module (200) to determine the User (100) medical records (2100), other reported habits and conditions. The User (100) can utilize the Dialogue Module (300) to tell the MyDoctor Platform (600) their emotional and psychological issues. For example, the User (100) can be trembling with a shaky voice, shortness of breath that is impacting their speech, sweating and complaining of chest pain and choking. The Diagnostic Module (400) may immediately conclude this is an anxiety or panic attack and respond to the User (100) to perform self-therapy of breathing exercises, at the same time provide positive encouragement with a soothing voice and music or natural sounds. In a more severe situation, a User (100) can speak to the MyDoctor Platform (600) that they want to commit suicide. The MyDoctor Platform (600) may immediately notify emergency services and distract the User (100) with questions, positive encouragement to give emergency services time to reach the User (100).

The platform may conduct analysis of the physical condition which in many cases could affect the Psychology of the person. The Diagnostic Module may be employed to trace the possibility of the physical conditions such as imbalance of chemicals that could affect the psychology. A similar diagnostic table to the physical diagnostic table may be triggered when the patient complaint is relevant to sentimental concerns. After the diagnostic, and conducting certain predefined physiological tests the system may trigger the Treatment Sub-Module where various treatment plans may be determined. In another instance, the User (100) can have long term psychological therapy utilizing the Psychological Therapy Module (1700). The therapy could be self-improvement, building confidence, overcoming fear, listening to an audio book or simply to speak to the MyDoctor Platform (600) to offload stress and peace of mind. The MyDoctor Platform (600) may collect this User (100) data and through machine learning track the User (100) improvement and adjust therapy sessions.

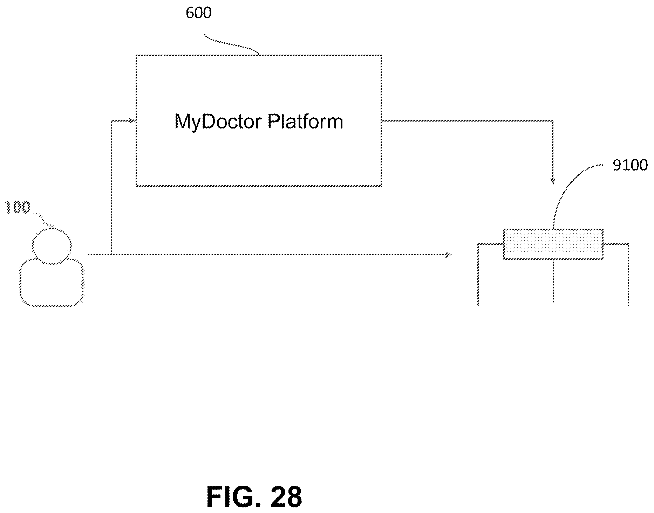

As seen in FIG. 28, in some embodiments, the MyDoctor platform (600) may further comprise a drone (9100) comprising a built-in GPS unit comprising a plurality of microprocessors and a plurality of antennas for directly receiving data sent by satellites through dedicated RF frequencies. The drone (9100) may be capable of delivering small objects back and forth between the user and an external source. For example, the drone (9100) may be capable of delivering a test sample from the user to the external source, delivering a prescription from the external source to the user, etc. The drone (9100) may implement an Internet connection and mapping software to increase the accuracy of drone deliveries or pickups. The drone (9100) may be capable of interacting directly with any module of the MyDoctor platform (600), capable of being programmed by the user separate from the MyDoctor platform (600), or both.

Although there has been shown and described the preferred embodiment of the present invention, it may be readily apparent to those skilled in the art that modifications may be made thereto which do not exceed the scope of the appended claims. Therefore, the scope of the invention is only to be limited by the following claims. In some embodiments, the figures presented in this patent application are drawn to scale, including the angles, ratios of dimensions, etc. In some embodiments, the figures are representative only and the claims are not limited by the dimensions of the figures. In some embodiments, descriptions of the inventions described herein using the phrase "comprising" includes embodiments that could be described as "consisting essentially of" or "consisting of", and as such the written description requirement for claiming one or more embodiments of the present invention using the phrase "consisting essentially of" or "consisting of" is met.

The reference numbers recited in the below claims are solely for ease of examination of this patent application, and are exemplary, and are not intended in any way to limit the scope of the claims to the particular features having the corresponding reference numbers in the drawings.

## Claims

1. A virtual physician platform system (600) for compiling a user's medical history from a plurality of sources, using the medical history to diagnose a medical issue of a user, the platform system (600) comprising:
I. a means for containing and executing a virtual physician software, the virtual physician software comprising:
a. an external communicator module (900) for gathering information pertaining to the user's medical history from a plurality of sources, the external communicator module (900) comprising:
i. a data encryption module (910) comprising instructions for:
1. accepting data from a source, and
2. encrypting the data into a standard format,
wherein the data encryption module (900) is capable of accepting data directly from the user, or from a plurality of external medical sources (930);
ii. a data compression module (920) comprising instructions for:
1. accepting encrypted data from the data encryption module (910),
2. compressing the encrypted data into compressed data, and
3. transmitting the compressed data to an external storage component (9000) or to the plurality of external medical sources (930);
b. a database of medical records (2100) received from the plurality of external medical sources (930) comprising:
i. a medical history of the user,
ii. a list of pre-existing conditions of the user, and
iii. a list of medications currently taken by the user,
wherein data found in the database of medical records (2100) is received from the plurality of external medical sources (930), such that each medical record is converted to a standard format upon being received by the virtual physician software; and
c. a diagnostic module comprising instructions for:
i. accessing a disease database of disease symptoms mapped to possible causes,
ii. accepting a list of user symptoms from the user,
iii. generating a first table (410) mapping each user symptom to all possible causes for the user symptom,
iv. generating a second table displaying all possible causes (450) that share every user symptom,
v. accepting data from the database of medical records (2100),
vi. determining, based on the second table, for each possible cause, a probability weight by dividing a number of symptoms matched between the user symptoms and symptoms of the possible cause by a total number of user symptoms,
vii. ranking all possible causes (450) based on the probability weights to generate a probability table,
viii. accessing a test repository of possible causes mapped to tests necessary for confirming possible causes,
ix. requesting a plurality of tests from the user to better determine the possible cause, wherein the plurality of tests comprises tests from the test repository necessary for confirming the possible cause,
x. accepting a set of test results from the user, and
xi. automatically determining, based on the data from the database of medical records (2100), the list of user symptoms, and the set of test results, a medical condition from the possible causes;
d. a treatment module (700) comprising instructions for:
i. selecting, from a Master Treatment table, a treatment plan for the medical condition generated by the diagnostic module (400), wherein the treatment plan comprises a prescription and a plurality of recommendations;
ii. adjusting the treatment plan to suit the user such that the prescription and the plurality of recommendations align with the user's medical history; and
iii. providing to the user a pharmacy capable of providing the prescription, pricing information for the prescription, and the plurality of recommendations;
wherein the external communicator module (900) further comprises instructions for autonomously issuing a request for the prescription determined by the treatment module (700) to the pharmacy provided by the treatment module (700);
e. a case monitoring module (800) comprising instructions for:
i. monitoring the user's health and recovery as the user carries out the treatment (475) determined by the treatment module (700),
ii. reminding the user to carry out a prescription related to the treatment (475),
iii. comparing an expected recovery time to an actual recovery time;
iv. changing the prescription into an updated prescription if the expected recovery time does not match the actual recovery time; and
v. adjusting the expected recovery time based on the updated prescription;
wherein each module of the software is capable of transmitting data to all other modules of the software; and
II. the disease database comprising disease symptoms mapped to possible diseases; and
III. the external storage component (9000).

2. The virtual physician platform system (600) of claim 1 further comprising testing equipment for allowing the user to carry out a plurality of tests.

3. The virtual physician platform system (600) of claim 2 further comprising a test analyzer module (500) for accepting tests from the user through use of the testing equipment and processing the tests to generate the set of test results to aid the diagnosis platform system in generating the possible cause.

4. The virtual physician platform system (600) of claim 1, wherein the external communicator module (900) further comprises instructions for ordering the prescription to be delivered to the user.

5. The virtual physician platform system (600) of claim 1 further comprising a nursing services module (1000) for allowing the user to request a registered nurse or licensed health care provider to aid the user with medical issues, testing, treatment or elderly and disability care from a list of registered nurses.

6. The virtual physician platform system (600) of claim 1 further comprising a dietitian module (1100) for keeping track of the user's dietary habits as data to be added to the database of medical records (2100) and for aiding the user in maintaining a healthy diet, losing weight and to prevent medical interactions in accordance with the treatment determined by the platform system.

7. The virtual physician platform system (600) of claim 1 further comprising a central research module (1200) for communicating with a research server comprising a database of data recorded by a plurality of instances of the platform system to transmit and receive test results, diagnoses, and recovery times.

8. The virtual physician platform system (600) of claim 7 further comprising a user account module (200) comprising instructions for:
A. storing general information (2000) of a user,
B. receiving medical records (2100) of the user from the external communicator module (900),
C. storing the medical records (2100) of the user,
D. storing reported conditions (2200) of the user,
E. transmitting the reported conditions (2200) of the user to the central research module (1200),
F. storing user habits (2300) of the user, and
G. transmitting a payment to an external payment source through the use of a payment gateway (2400).

9. The virtual physician platform system (600) of claim 8 further comprising a wellbeing module (1300) comprising instructions for:
A. requesting, based on a data science algorithm applied to information from the user account module (200), one or more screening tests, and
B. recommending, based on results of the one or more screening tests, one or more supplements.

10. The virtual physician platform system (600) of claim 8 further comprising a physical activity module (1400) comprising instructions for:
A. tracking physical activity of the user,
B. receiving a desired physical activity result from the user, and
C. providing, based on information from the user account module (200), feedback on the physical activity of the user to aid the user in achieving the desired physical activity result.

11. The virtual physician platform system (600) of claim 1 further comprising a breathing apparatus module (1500) comprising:
A. a ventilation device for aiding in the user's breathing, and
B. a nebulizer for delivering medicine to a set of lungs of the user.

12. The virtual physician platform system (600) of claim 1 further comprising a personal vital signs monitor configured to detect vital signs of the user and a defibrillator module (1600) comprising a defibrillation device for electrically stimulating a heart of the user and comprising instructions for:
A. providing instructions on how to use the defibrillation device, and
B. contacting emergency medical aid upon the personal vital signs monitor detecting vital signs requiring immediate attention.

13. The virtual physician platform system (600) of claim 1 further comprising a psychological therapy module (1700) comprising instructions for:
A. determining, based on a tone of voice of the user, whether the user is in distress, in pain, anxious, depressed, or angry,
B. contacting emergency medical aid upon the user expressing a desire to commit suicide, and
C. providing long-term psychological therapy to the user.

## Patentansprüche

1. Ein virtuelles Arztplattformsystem (600) zum Zusammenstellen der Krankengeschichte eines Benutzers aus einer Vielzahl von Quellen, wobei die Krankengeschichte zur Diagnose eines medizinischen Problems eines Benutzers verwendet wird, wobei das Plattformsystem (600) umfasst:
I. ein Mittel zum Enthalten und Ausführen einer virtuellen Arztsoftware, wobei die virtuelle Arztsoftware Folgendes umfasst:
a. ein externes Kommunikationsmodul (900) zum Sammeln von Informationen über die Krankengeschichte des Benutzers aus einer Vielzahl von Quellen, wobei das externe Kommunikationsmodul (900) umfasst:
i. ein Datenverschlüsselungsmodul (910) umfassend Anweisungen zum:
1. Annehmen von Daten von einer Quelle und
2. Verschlüsseln der Daten in ein Standardformat,
wobei das Datenverschlüsselungsmodul (910) in der Lage ist, Daten direkt vom Benutzer oder von einer Vielzahl von externen medizinischen Quellen (930) anzunehmen;
ii. ein Datenkomprimierungsmodul (920) umfassend Anweisungen zum:
1. Annehmen verschlüsselter Daten von dem Datenverschlüsselungsmodul (910),
2. Komprimieren der verschlüsselten Daten zu komprimierten Daten und
3. Übertragen der komprimierten Daten an eine externe Speicherkomponente (9000) oder an die Vielzahl externer medizinischer Quellen (930);
b. eine Datenbank medizinischer Unterlagen (2100), die von der Vielzahl von externen medizinischen Quellen (930) empfangen wurden, umfassend:
i. eine Krankengeschichte des Benutzers,
ii. eine Liste von Vorerkrankungen des Benutzers und
iii. eine Liste der derzeit vom Benutzer eingenommenen Medikamente,
wobei Daten, die sich in der Datenbank mit medizinischen Unterlagen (2100) befinden, von der Vielzahl von externen medizinischen Quellen (930) empfangen werden, sodass alle medizinischen Unterlagen bei Empfang durch die virtuelle Arztsoftware in ein Standardformat konvertiert werden; und
c. ein Diagnosemodul umfassend Anweisungen zum:
i. Zugreifen auf eine Krankheitsdatenbank mit Krankheitssymptomen, die möglichen Ursachen zugeordnet sind,
ii. Annehmen einer Liste von Benutzersymptomen vom Benutzer,
iii. Erstellen einer ersten Tabelle (410), die jedes Benutzersymptom allen möglichen Ursachen für das Benutzersymptom zuordnet,
iv. Erstellen einer zweiten Tabelle, die alle möglichen Ursachen (450) angibt, die alle Benutzersymptome gemeinsam haben,
v. Annehmen von Daten aus der Datenbank medizinischer Unterlagen (2100),
vi. Bestimmen einer Wahrscheinlichkeitsgewichtung für jede mögliche Ursache auf der Grundlage der zweiten Tabelle, indem eine Anzahl von Symptomen, die zwischen den Symptomen des Benutzers und den Symptomen der möglichen Ursache übereinstimmen, durch eine Gesamtzahl von Symptomen des Benutzers dividiert wird,
vii. Ordnen aller möglichen Ursachen (450) auf der Grundlage der Wahrscheinlichkeitsgewichtungen, um eine Wahrscheinlichkeitstabelle zu erstellen,
viii. Zugreifen auf ein Test-Repository möglicher Ursachen, die Tests zugeordnet sind, die zur Bestätigung möglicher Ursachen erforderlich sind,
ix. Anfordern einer Vielzahl von Tests vom Benutzer, um die mögliche Ursache besser zu bestimmen, wobei die Vielzahl von Tests Tests aus dem Test-Repository umfasst, die zur Bestätigung der möglichen Ursache erforderlich sind,
x. Annehmen einer Reihe von Testergebnissen vom Benutzer und
xi. automatisches Bestimmen einer Erkrankung aus den möglichen Ursachen auf der Grundlage der Daten aus der Datenbank medizinischer Unterlagen (2100), der Liste der Benutzersymptome und der Reihe von Testergebnissen;
d. ein Behandlungsmodul (700) umfassend Anweisungen zum:
i. Auswählen eines vom Diagnosemodul (400) erstellten Behandlungsplans für die Erkrankung aus einer Master-Behandlungstabelle,
wobei der Behandlungsplan eine Verschreibung und eine Vielzahl von Empfehlungen umfasst;
ii. Anpassen des Behandlungsplans an den Benutzer, sodass die Verschreibung und die Vielzahl von Empfehlungen die Krankengeschichte des Benutzers berücksichtigen; und
iii. Bereitstellen einer Apotheke für den Benutzer, die in der Lage ist, die Verschreibung bereitzustellen, von Preisinformationen für die Verschreibung sowie der Vielzahl von Empfehlungen;
wobei das externe Kommunikationsmodul (900) ferner Anweisungen umfasst, um autonom eine Anfrage für die vom Behandlungsmodul (700) bestimmte Verschreibung an die vom Behandlungsmodul (700) bereitgestellte Apotheke zu senden;
e. ein Fallüberwachungsmodul (800) umfassend Anweisungen zum
i. Überwachen der Gesundheit und der Genesung des Benutzers, während der Benutzer die vom Behandlungsmodul (700) bestimmte Behandlung (475) durchführt,
ii. Erinnern des Benutzers an die Durchführung einer mit der Behandlung (475) verbundenen Verschreibung,
iii. Vergleichen einer erwarteten Genesungszeit mit einer tatsächlichen Genesungszeit;
iv. Ändern der Verschreibung in eine aktualisierte Verschreibung, wenn die erwartete Genesungszeit nicht mit der tatsächlichen Genesungszeit übereinstimmt; und
v. Anpassen der erwarteten Genesungszeit auf der Grundlage der aktualisierten Verschreibung;
wobei jedes Modul der Software in der Lage ist, Daten an alle anderen Module der Software zu übertragen; und
II. die Krankheitsdatenbank, die Krankheitssymptome umfasst, die möglichen Krankheiten zugeordnet sind; und
III. die externe Speicherkomponente (9000).

2. Virtuelles Arztplattformsystem (600) nach Anspruch 1 ferner umfassend Testausrüstung, die es dem Benutzer erlaubt, eine Vielzahl von Tests durchzuführen.

3. Virtuelles Arztplattformsystem (600) nach Anspruch 2 ferner umfassend ein Testanalysemodul (500) zum Annehmen von Tests vom Benutzer unter Verwendung der Testausrüstung und zum Verarbeiten der Tests, um den Satz von Testergebnissen zu erstellen, der das Diagnoseplattformsystem beim Erstellen der möglichen Ursache unterstützt.

4. Virtuelles Arztplattformsystem (600) nach Anspruch 1, wobei das externe Kommunikationsmodul (900) ferner Anweisungen zum Bestellen der an den Benutzer zu liefernden Verschreibung umfasst.

5. Virtuelles Arztplattformsystem (600) nach Anspruch 1 ferner umfassend ein Pflegedienstmodul (1000), mit dem der Benutzer registriertes Pflegepersonal oder einen zugelassenen Gesundheitsdienstleister aus einer Liste von registriertem Pflegepersonal anfordern kann, um den Benutzer bei medizinischen Problemen, Tests, Behandlungen oder der Pflege älterer und behinderter Menschen zu unterstützen.

6. Virtuelles Arztplattformsystem (600) nach Anspruch 1 ferner umfassend ein Ernährungsberatermodul (1100) zum Nachverfolgen der Ernährungsgewohnheiten des Benutzers als Daten, die zur Datenbank mit medizinischen Unterlagen (2100) hinzugefügt werden sollen, und zum Unterstützen des Benutzers bei der Aufrechterhaltung einer gesunden Ernährung, beim Abnehmen und beim Verhindern medizinischer Wechselwirkungen gemäß der vom Plattformsystem bestimmten Behandlung.

7. Virtuelles Arztplattformsystem (600) nach Anspruch 1 ferner umfassend ein zentrales Forschungsmodul (1200) zur Kommunikation mit einem Forschungsserver, der eine Datenbank mit Daten umfasst, die von einer Vielzahl von Instanzen des Plattformsystems aufgezeichnet wurden, um Testergebnisse, Diagnosen und Genesungszeiten zu übertragen und zu empfangen.

8. Virtuelles Arztplattformsystem (600) nach Anspruch 7 ferner umfassend ein Benutzerkontomodul (200) umfassend Anweisungen zum:
A. Speichern allgemeiner Informationen (2000) eines Benutzers,
B. Empfangen medizinischer Unterlagen (2100) des Benutzers vom externen Kommunikationsmodul (900),
C. Speichern der medizinischen Unterlagen (2100) des Benutzers,
D. Speichern gemeldeter Erkrankungen (2200) des Benutzers,
E. Übertragen der gemeldeten Erkrankungen (2200) des Benutzers an das zentrale Forschungsmodul (1200),
F. Speichern der Benutzergewohnheiten (2300) des Benutzers und
G. Übertragen einer Zahlung an eine externe Zahlungsquelle unter Verwendung eines Zahlungsgateways (2400).

9. Virtuelles Arztplattformsystem (600) nach Anspruch 8 ferner umfassend ein Wellbeing-Modul (1300) umfassend Anweisungen zum:
A. Anfordern eines oder mehrerer Screening-Tests auf der Grundlage eines Datenwissenschaftsalgorithmus, der auf Informationen aus dem Benutzerkontenmodul (200) angewendet wird, und
B. Empfehlen eines oder mehrerer Nahrungsergänzungsmittel auf der Grundlage der Ergebnisse des einen oder der mehreren Screening-Tests.

10. Virtuelles Arztplattformsystem (600) nach Anspruch 8 ferner umfassend ein Körperaktivitätsmodul (1400) umfassend Anweisungen zum:
A. Nachverfolgen der Körperaktivität des Benutzers,
B. Empfangen eines gewünschten Körperaktivitätsergebnisses vom Benutzer und
C. Bereitstellen von Feedback zur Körperaktivität des Benutzers auf der Grundlage von Informationen aus dem Benutzerkontomodul (200), um den Benutzer beim Erreichen des gewünschten Körperaktivitätsergebnisses zu unterstützen.

11. Virtuelles Arztplattformsystem (600) nach Anspruch 1 ferner umfassend ein Atemgerätmodul (1500), umfassend:
A. eine Beatmungsvorrichtung zur Unterstützung der Atmung des Benutzers und
B. einen Vernebler zur Abgabe von Medikamenten an die Lunge des Benutzers.

12. Virtuelles Arztplattformsystem (600) nach Anspruch 1 ferner umfassend einen persönlichen Vitalparameter-Monitor, der so ausgebildet ist, dass er die Vitalparameter des Benutzers erfasst, und ein Defibrillatormodul (1600), das eine Defibrillationsvorrichtung zur elektrischen Stimulation des Herzens des Benutzers umfasst und Anweisungen umfasst für:
A. die Bereitstellung von Anweisungen zur Verwendung der Defibrillationsvorrichtung und
B. die Kontaktaufnahme mit dem medizinischen Notdienst, wenn der persönliche Vitalparameter-Monitor Vitalparameter erfasst, die sofortige Aufmerksamkeit erfordern.

13. Virtuelles Arztplattformsystem (600) nach Anspruch 1 ferner umfassend ein Psychotherapiemodul (1700) umfassend Anweisungen zum:
A. Bestimmen, basierend auf dem Tonfall des Benutzers, ob der Benutzer in Not, unter Schmerzen, ängstlich, depressiv oder wütend ist,
B. Kontaktieren des medizinischen Notdienstes, wenn der Benutzer den Wunsch äußert, Suizid zu begehen, und
C. Bereitstellen einer langfristigen Psychotherapie für den Benutzer.

## Revendications

1. Système de plateforme de médecin virtuel (600) destiné à compiler les antécédents médicaux d'un utilisateur à partir d'une pluralité de sources, en utilisant les antécédents médicaux pour diagnostiquer un problème médical d'un utilisateur, le système de plateforme (600) comprenant :
I. un moyen pour contenir et exécuter un logiciel de médecin virtuel, le logiciel de médecin virtuel comprenant :
a. un module de communication externe (900) pour recueillir des informations relatives aux antécédents médicaux de l'utilisateur à partir d'une pluralité de sources, le module de communication externe (900) comprenant :
i. un module de cryptage de données (910) comprenant des instructions pour :
1. accepter des données provenant d'une source, et
2. crypter les données dans un format standard,
dans lequel le module de cryptage de données (900) est capable d'accepter des données directement de l'utilisateur, ou d'une pluralité de sources médicales externes (930) ;
ii. un module de compression de données (920) comprenant des instructions pour:
1. accepter des données cryptées provenant du module de cryptage de données (910),
2. compresser les données cryptées en données compressées, et
3. transmettre les données compressées à un composant de stockage externe (9000) ou à la pluralité de sources médicales externes (930) ;
b. base de données de dossiers médicaux (2100) reçus de la pluralité de sources médicales externes (930) comprenant :
i. les antécédents médicaux de l'utilisateur,
ii. une liste des conditions préexistantes de l'utilisateur, et
iii. une liste des médicaments actuellement pris par l'utilisateur,
dans lequel les données trouvées dans la base de données des dossiers médicaux (2100) sont reçues à partir de la pluralité de sources médicales externes (930), de telle sorte que chaque dossier médical est converti en un format standard dès sa réception par le logiciel de médecin virtuel ; et
c. un module de diagnostic comprenant des instructions pour :
i. accéder à une base de données de maladies répertoriant les symptômes associés à des causes possibles,
ii. accepter une liste de symptômes fournie par l'utilisateur,
iii. générer un premier tableau (410) associant chaque symptôme de l'utilisateur à toutes les causes possibles de ce symptôme,
iv. générer un deuxième tableau affichant toutes les causes possibles (450) qui partagent chaque symptôme de l'utilisateur,
v. accepter les données provenant de la base de données des dossiers médicaux (2100),
vi. déterminer, sur la base du deuxième tableau, pour chaque cause possible, une pondération de probabilité en divisant le nombre de symptômes correspondant entre les symptômes de l'utilisateur et les symptômes de la cause possible par le nombre total de symptômes de l'utilisateur,
vii. classer toutes les causes possibles (450) en fonction des pondérations de probabilité afin de générer un tableau de probabilités,
viii. accéder à un référentiel de tests des causes possibles associées aux tests nécessaires pour confirmer les causes possibles,
ix. demander à l'utilisateur d'effectuer plusieurs tests afin de mieux déterminer la cause possible, lesdits tests comprenant les tests du référentiel de tests nécessaires pour confirmer la cause possible,
x. accepter un ensemble de résultats de tests provenant de l'utilisateur, et
xi. déterminer automatiquement, sur la base des données provenant de la base de données de dossiers médicaux (2100), de la liste des symptômes de l'utilisateur et de l'ensemble des résultats de tests, un état médical parmi les causes possibles ;
d. un module de traitement (700) comprenant des instructions pour :
i. sélectionner, à partir d'un tableau de traitement maître, un plan de traitement pour l'affection médicale générée par le module de diagnostic (400), dans lequel le plan de traitement comprend une ordonnance et une pluralité de recommandations ;
ii. adapter le plan de traitement à l'utilisateur de manière à ce que l'ordonnance et les recommandations correspondent à ses antécédents médicaux ; et
iii. fournir à l'utilisateur une pharmacie capable de délivrer l'ordonnance, des informations sur le prix de l'ordonnance et plusieurs recommandations ;
dans lequel le module de communication externe (900) comprend en outre des instructions pour émettre de manière autonome une demande pour l'ordonnance déterminée par le module de traitement (700) à la pharmacie fournie par le module de traitement (700) ;
e. un module de surveillance de cas (800) comprenant des instructions pour
i. surveiller la santé et le rétablissement de l'utilisateur pendant que celui-ci suit le traitement (475) déterminé par le module de traitement (700),
ii. rappeler à l'utilisateur de suivre une ordonnance relative au traitement (475),
iii. comparer un temps de récupération prévu à un temps de récupération réel ;
iv. modifier l'ordonnance pour la mettre à jour si le temps de récupération prévu ne correspond pas au temps de récupération réel ; et
v. ajuster le temps de récupération prévu en fonction de l'ordonnance mise à jour ;
dans lequel chaque module du logiciel est capable de transmettre des données à tous les autres modules du logiciel ; et
II. la base de données sur les maladies comprenant les symptômes de maladies associés à des maladies possibles ; et
III. le composant de stockage externe (9000).

2. Système de plateforme de médecin virtuel (600) selon la revendication 1, comprenant en outre un équipement de test permettant à l'utilisateur d'effectuer une pluralité de tests.

3. Système de plateforme de médecin virtuel (600) selon la revendication 2, comprenant en outre un module d'analyseur de tests (500) destiné à accepter les tests de l'utilisateur à l'aide de l'équipement de test et à traiter les tests afin de générer l'ensemble de résultats de tests pour aider le système de plateforme de diagnostic à générer la cause possible.

4. Système de plateforme de médecin virtuel (600) selon la revendication 1, dans lequel le module de communication externe (900) comprend en outre des instructions pour commander la livraison de l'ordonnance à l'utilisateur.

5. Système de plateforme de médecin virtuel (600) selon la revendication 1, comprenant en outre un module de services infirmiers (1000) permettant à l'utilisateur de demander à une infirmière diplômée ou à un professionnel de santé agréé de l'aider pour des questions médicales, des tests, des traitements ou des soins aux personnes âgées et handicapées à partir d'une liste d'infirmières diplômées.

6. Système de plateforme de médecin virtuel (600) selon la revendication 1, comprenant en outre un module diététicien (1100) destiné à suivre les habitudes alimentaires de l'utilisateur sous forme de données à ajouter à la base de données des dossiers médicaux (2100) et à aider l'utilisateur à maintenir une alimentation saine, à perdre du poids et à prévenir les interactions médicales conformément au traitement déterminé par le système de plateforme.

7. Système de plateforme de médecin virtuel (600) selon la revendication 1, comprenant en outre un module de recherche central (1200) destiné à communiquer avec un serveur de recherche comprenant une base de données de données enregistrées par une pluralité d'instances du système de plateforme afin de transmettre et de recevoir des résultats de tests, des diagnostics et des délais de guérison.

8. Système de plateforme de médecin virtuel (600) selon la revendication 7, comprenant en outre un module de compte utilisateur (200) comprenant des instructions pour :
A. stocker des informations générales (2000) sur un utilisateur,
B. recevoir les dossiers médicaux (2100) de l'utilisateur à partir du module de communication externe (900),
C. stocker les dossiers médicaux (2100) de l'utilisateur,
D. stocker les conditions signalées (2200) de l'utilisateur,
E. transmettre les conditions signalées (2200) de l'utilisateur au module de recherche central (1200),
F. stocker les habitudes (2300) de l'utilisateur, et
G. transmettre un paiement à une source de paiement externe via l'utilisation d'une passerelle de paiement (2400).

9. Système de plateforme de médecin virtuel (600) selon la revendication 8, comprenant en outre un module de bien-être (1300) comprenant des instructions pour :
A. demander, sur la base d'un algorithme de science des données appliqué aux informations provenant du module de compte utilisateur (200), un ou plusieurs tests de dépistage, et
B. recommander, sur la base des résultats d'un ou plusieurs tests de dépistage, un ou plusieurs compléments alimentaires.

10. Système de plateforme de médecin virtuel (600) selon la revendication 8, comprenant en outre un module d'activité physique (1400) comprenant des instructions pour :
A. suivre l'activité physique de l'utilisateur,
B. recevoir un résultat d'activité physique souhaité de la part de l'utilisateur, et
C. fournir, sur la base des informations provenant du module de compte utilisateur (200), un retour d'information sur l'activité physique de l'utilisateur afin d'aider celui-ci à atteindre le résultat souhaité en matière d'activité physique.

11. Système de plateforme de médecin virtuel (600) selon la revendication 1, comprenant en outre un module d'appareil respiratoire (1500) comprenant :
A. un dispositif de ventilation pour aider la respiration de l'utilisateur, et
B. un nébuliseur pour administrer un médicament à un ensemble de poumons de l'utilisateur.

12. Système de plateforme de médecin virtuel (600) selon la revendication 1, comprenant en outre un moniteur personnel de signes vitaux configuré pour détecter les signes vitaux de l'utilisateur et un module défibrillateur (1600) comprenant un dispositif de défibrillation pour stimuler électriquement le cœur de l'utilisateur et comprenant des instructions pour :
A. fournir des instructions sur l'utilisation du défibrillateur, et
B. contacter les secours médicaux d'urgence lorsque le moniteur des signes vitaux détecte des signes vitaux nécessitant une attention immédiate.

13. Système de plateforme de médecin virtuel (600) selon la revendication 1, comprenant en outre un module de thérapie psychologique (1700) comprenant des instructions pour :
A. déterminer, en fonction du ton de voix de l'utilisateur, si celui-ci est en détresse, souffre, est anxieux, déprimé ou en colère,
B. contacter les secours médicaux d'urgence lorsque l'utilisateur exprime son désir de se suicider, et
C. fournir une thérapie psychologique à long terme à l'utilisateur.
